# EUROPEAN PATENT APPLICATION

(11) **EP 4 769 431 A2**
(43) Date of publication of application: **01.07.2026**
(21) Application number: 26164084.1
(22) Date of filing: 17.12.2021
(51) Int. Cl.: G16H 50/70

(54) **RESPIRATORY SYSTEM AND METHOD**

(30) Priority: 18.12.2020 US 202063127860 P
(62) Divisional of application: 21905962.3
(71) Applicant: Fisher & Paykel Healthcare Limited, East Tamaki, Auckland 2013 (NZ)
(72) Inventor: GRAY, Nathanael Charles, 2013 Auckland (NZ); WILSON, Matthew Robert, 2013 Auckland (NZ); OSBORNE, Hamish Adrian, 2013 Auckland (NZ); PAYTON, Matthew Jon, 2013 Auckland (NZ); CHEUNG, Man Kit Jacky, 2013 Auckland (NZ)
(74) Representative: Forresters IP LLP

(57) **Abstract**

This present disclosure relates to a system and method for providing a flow of respiratory gases to a patient. The system includes a controller, a flow modulator, and one or more sensors, wherein the controller is configured to control the flow modulator to modulate the flow of respiratory gases in response to determined pressure in the system exceeding or not exceeding a pressure threshold value.

## Description

### Technical Field

This disclosure relates to a system and method for controlling a flow of respiratory gases to a patient.

In particular, but not exclusively, the system and method controls the flow of respiratory gases to the patient in response to a change in the system pressure downstream of a flow modulator providing the flow of respiratory gases.

### Background of Invention

Patients may lose respiratory function during anaesthesia, or sedation, or more generally during certain medical procedures. Prior to a medical procedure a patient may be pre-oxygenated by a medical professional to provide a reservoir of oxygen saturation, and this pre-oxygenation is generally carried out with a bag ventilator and a face mask. Once under general anaesthesia, patients must be intubated to ventilate the patient. In some cases, intubation is completed in 30 to 60 seconds, but in other cases, particularly if the patient's airway is difficult to traverse (for example, due to cancer, severe injury, obesity or spasm of the neck muscles), intubation will take significantly longer. While pre-oxygenation provides a buffer against declines in oxygen saturation, for long intubation procedures, it is necessary to interrupt the intubation process and reapply the face mask to increase the patient's oxygen saturation to adequate levels. The interruption of the intubation process may happen several times for difficult intubation processes, which is time consuming and puts the patient at severe health risk. After approximately three attempts at intubation the medical procedure will be abandoned.

In procedures where multiple respiratory support systems are required, there may be a concern that the combination(s) of support systems could cause excessive pressure delivery (for example when a cannula is in place on a patient and an anaesthetist wishes to deliver respiratory support through a mask applied over the top of the cannula).

Furthermore, switching between different support systems may be time consuming or difficult. It may therefore be desirable to have a configuration that allows easy interchange between respiratory support systems, for example support via high flow and respiratory support via a face mask and bag or anaesthesia machine. It may also be desirable to allow gas flows to be quickly and easily turned off or reduced.

A reference herein to a patent document, or any other matter identified as prior art, is not to be taken as an admission that the document or other matter was known or that the information it contains was part of the common general knowledge as at the priority date of any of the claims.

### Summary of Invention

In one aspect of the present disclosure, there is provided a respiratory system for providing a flow of respiratory gases to a patient, the system comprising: a flow modulator; a controller configured to receive an input of a flow rate and a pressure of the flow of respiratory gases in the system, and configured to: control the flow modulator to provide the flow of respiratory gases at a target flow rate to a patient; and control the flow modulator to modulate the flow of respiratory gases at a target pressure when the pressure of the flow of respiratory gases in the system meets or exceeds a pressure threshold value corresponding to that target flow rate.

In some embodiments, the target pressure comprises the pressure threshold value corresponding to the target flow rate or a pressure threshold value corresponding to a flow rate less than the target flow rate.

In some embodiments, the controller is configured to control the flow modulator to modulate the flow of respiratory gases to the target pressure when the flow rate of the flow of respiratory gases is less than the target flow rate.

In some embodiments, the controller is configured to control the flow modulator to modulate the flow of respiratory gases to the target flow rate when the flow rate of the flow of respiratory gases is above the target flow rate.

In some embodiments, the controller is configured to control the flow modulator to the target pressure when the pressure of the flow of respiratory gases exceeds the pressure threshold value corresponding to the target flow rate or a pressure threshold value corresponding to flow rates less than the target flow rate.

In some embodiments, when the system begins operation from an 'off' or dormant state, the controller is configured to monitor the flow rate of the flow of respiratory gases in the system and is configured to control the flow modulator to modulate the flow of respiratory gases to the target flow rate. In some embodiments, the target flow rate changes over time to increase from a current value to a flow rate set point.

In some embodiments, the target flow rate is a flow rate set point. The flow rate set point may determined by a user of the respiratory system. In some embodiments, the flow rate set point may be set by a user providing an input to the controller.

In some embodiments, the controller comprises a flow rate controller providing a flow rate control output and a pressure controller providing a pressure control output, wherein the control input to the flow modulator is the minimum of the flow rate control output and the pressure control output.

In some embodiments, the flow modulator comprises a blower, and the flow rate control output and pressure control output comprise an angular velocity of the blower.

In some embodiments, the flow modulator comprises a proportional valve, and the flow rate control output and pressure control output comprise a size of a flow path restriction through the proportional valve.

In some embodiments, the controller is configured to receive a flow rate value indicative of flow rate of respiratory gases provided to the patient.

In some embodiments, the controller may be operable in a first control mode when the flow rate is above the target flow rate to control the flow rate to the target flow rate, and the controller is operable in a second control mode when the pressure of the flow of respiratory gases is above the pressure threshold value corresponding to the target flow rate or is above a or the pressure threshold value corresponding to a flow rate less than the target flow rate, to control the pressure to the target pressure.

In some embodiments, the respiratory system comprises one or more flow rate sensors configured to sense a flow rate of the flow of respiratory gases in the system to the patient

In some embodiments, the respiratory system comprises one or more pressure sensors configured to sense a pressure of the flow of respiratory gases in the system to the patient.

In some embodiments, the controller is further configured to receive an input indicative of the flow rate of the flow of respiratory gases in the system from the one or more flow rate sensors, and an input indicative of the pressure of the flow of respiratory gases in the system from the one or more pressure sensors.

In some embodiments, the respiratory system comprises a delivery conduit for providing a flow of gases from the flow modulator to a patient interface, wherein the patient interface is configured to deliver the flow of respiratory gases to the patient.

In some embodiments, patient interface comprises a nasal cannula, optionally a non-sealing nasal cannula.

In some embodiments, the patient interface comprises a gases delivery side arm in fluid communication with the delivery conduit, a manifold provided at an end of the gases delivery side arm, and one or more nasal elements extending from the manifold, the one or more nasal element configured to provide the flow of respiratory gases to one or more nares of the patient, wherein the gases delivery side member comprises a collapsible portion.

The collapsible portion may be operable in a first configuration in which the collapsible portion is in a substantially open condition, and in a second configuration in which the collapsible portion is in a substantially closed condition.

In some embodiments, when the controller controls the flow modulator to the target pressure, flow to the patient is reduced to a reduced flow rate which is: about 15L/min or less; or about 10L/min or less; or about 10L/min; or about 5L/min to about 10L/min or less than about 5L/min or 0L/min.

In some embodiments, the controller is configured to control the flow modulator to increase the flow of respiratory gases towards the target flow rate.

In some embodiments, the delivery conduit further comprises a delivery circuit and a patient breathing circuit disposed between the delivery circuit and the patient interface, and wherein the patient breathing circuit is connected to the delivery circuit by an outlet connector.

In some embodiments, the flow modulator comprises one or more of: a flow generator configured to be controlled by the controller to modulate the flow of respiratory gases to a patient; and a proportional valve configured to be controlled by the controller to modulate the flow of respiratory gases.

In some embodiments, the flow generator comprises a blower configured to be controlled by the controller to generate the flow of respiratory gases.

In some embodiments, the respiratory system comprises an Oxygen (O₂) pressure sensor configured to sense pressure in an O₂ delivery circuit of the respiratory system.

In some embodiments, the respiratory system comprises an Oxygen (O₂) flow rate sensor configured to sense flow rate of a flow of O₂ in an O₂ delivery circuit of the respiratory system. In some embodiments, a proportional valve may be disposed between the O₂ pressure sensor and the O₂ flow rate sensor in the O₂ delivery circuit.

In some embodiments, having a patient breathing circuit, the patient breathing circuit may be connected to the O₂ delivery circuit and an air delivery circuit and the patient breathing circuit and/or the patient interface may further comprise a patient pressure sensor and a patient flow rate sensor. In some embodiments, the flow modulator comprises a blower, and the blower may be disposed in the patient breathing circuit before the patient pressure sensor and the patient flow rate sensor.

In some embodiments, the controller is in operative communication with, or comprises, a memory component storing one or more of a function, a curve, a look up table or algorithm providing a relationship between the pressure threshold values and corresponding flow rates.

In some embodiments, the controller is configured to control the flow modulator to reduce the flow of respiratory gases by a variable rate of decrease.

In some embodiments, the relationship between the pressure threshold values and corresponding flow rates may be represented by a pressure limit curve or function defining a curve having a sigmoidal shape.

In some embodiments, the relationship between the pressure threshold values and corresponding flow rates comprises a first pressure region, a second pressure region, and a transition region disposed between the first pressure region and the second pressure region.

In some embodiments, the first pressure region may correspond to when the collapsible portion is substantially in the first configuration and the second region may correspond to when the collapsible portion is substantially in the second configuration.

In some embodiments, the first pressure region comprises pressure threshold values offset from baseline pressure values by a first pressure margin and, the second pressure region comprises pressure threshold values offset from baseline pressure values by a second pressure margin, whereby the first pressure margin is greater than the second pressure margin.

In some embodiments, the first and second margins provide an offset of the pressure threshold limit values in the first pressure region which is substantially parallel to an offset of the pressure threshold values in the second pressure region of the pressure limit curve, and the first pressure region and the second pressure region correspond to a minimum rate of change in the flow of respiratory gases.

In some embodiments, the gradient of the transition region of the pressure limit curve corresponds to a maximum rate of change in the flow of respiratory gases.

In some embodiments, the transition region of the pressure limit curve may be centred about a transition flow rate. In some embodiments, the controller is configured to control the flow modulator to achieve a flow of respiratory gases at the maximum rate of change when the pressure and the corresponding flow rate is in the transition region of the pressure limit curve.

In some embodiments, the first and the second pressure margins provide a margin for a temporary decrease in system pressure of the flow of respiratory gases from below the pressure threshold values without the controller controlling the flow modulator to increase the flow of respiratory gases.

Viewed from another aspect, the present invention provides a respiratory system for providing a flow of respiratory gases to a patient, the system comprising: a controller; and a flow modulator configured to be controlled by the controller to provide a flow of respiratory gases to a patient; wherein the controller is configured to control the flow modulator to a flow rate determined pressure set point; and wherein the controller is configured to control the flow modulator to limit the flow rate of the flow of respiratory gases up to and including a flow rate set point.

In some embodiments, the flow rate set point is set by a user of the respiratory system. In some embodiments, the flow rate set point may be set by a user providing an input to the controller.

In some embodiments, the system comprises a plurality of flow rate determined pressure set points, to which the controller is configured to control the flow modulator.

In some embodiments, the flow rate determined pressure set point is based on the flow rate of the flow of respiratory gases.

In some embodiments, the flow rate determined pressure set point is predetermined.

In some embodiments, the flow rate determined pressure set point when the flow rate of the flow of respiratory gases is at or close to the flow rate set point, is separated by a first margin from a normal system pressure value, wherein the flow rate determined pressure set point when the flow rate of the flow of respiratory gases at or close to 0L/min, is separated by a second margin from a normal system pressure value, wherein the first margin is greater than the second margin.

In some embodiments, the flow rate determined pressure set point when the flow rate of the flow of respiratory gases at or close to 0L/min, is 0cmH2O.

In some embodiments, the flow modulator comprises a blower and/or a proportional valve.

In some embodiments, the respiratory system further comprises one or more of a flow sensor, pressure sensor and a user interface.

In some embodiments, respiratory system further comprises a non-sealing nasal interface in fluid communication with the flow modulator and configured to provide the flow of respiratory gases to the patient.

In some embodiments, the non-sealing nasal interface comprises a gases delivery side arm, a manifold provided at an end of the gases delivery side arm, and one or more nasal elements extending from the manifold, the one or more nasal element configured to provide the flow of respiratory gases to one or more nares of the patient, wherein the gases delivery side member comprises a collapsible portion.

In some embodiments, the collapsible portion is operable in a first configuration in which the collapsible portion is in a substantially open condition, and in a second configuration in which the collapsible portion is in a substantially closed condition.

In some embodiments, the flow rate set point is more than 0 L/min, optionally more than 0 L/min to about 120 L/min, optionally between about 20 L/min to about 90 L/min, and optionally about 40 L/min to about 70 L/min.

In another aspect of the present disclosure, there is provided a respiratory system for providing a flow of respiratory gases to a patient, the system comprising: a controller; a flow modulator configured to be controlled by the controller to provide a flow of respiratory gases to a patient; wherein the controller is configured to: receive an input relating to a flow rate set point receive one or more pressure inputs indicative of system pressure corresponding to the flow of respiratory gases downstream of the flow modulator; receive one or more flow rate inputs indicative of the flow of respiratory gases downstream of the flow modulator; compare the received pressure and/or flow rate inputs with predetermined pressure threshold values for corresponding flow rates and the flow rate set point; control the flow modulator between two control modes, wherein the first control mode comprises controlling the flow modulator to provide the flow of respiratory gases at the flow rate set point when the system pressure is below the predetermined pressure threshold values for the corresponding flow rates; and the second control mode comprises controlling the flow modulator to modulate the flow of respiratory gases to a target pressure when the flow rate of the flow of respiratory gases is below the flow rate set point.

In another aspect of the present disclosure, there is provided a respiratory system for providing a flow of respiratory gases to a patient, the system comprising: a controller; a flow modulator configured to be controlled by the controller to provide a flow of respiratory gases to a patient; wherein the controller is configured to: receive an input indicative of the system pressure; compare the system pressure against one or more pressure threshold values for corresponding flow rates; control the flow modulator to reduce the system pressure in response to the system pressure meeting or exceeding the one or more pressure threshold values for the corresponding flow rates; and control the flow modulator to achieve a target flow rate in response to the system pressure not meeting or exceeding the pressure threshold values for the corresponding flow rates.

In another aspect of the present disclosure, there is provided a respiratory system for providing a flow of respiratory gases to a patient, the system comprising: a controller; and a flow modulator configured to be controlled by the controller to provide a flow of respiratory gases to a patient; wherein the controller is configured to control the flow modulator to a flow rate set point; and wherein the controller is configured to control the flow modulator to limit the pressure of the flow of respiratory gases up to and including a flow rate determined pressure limit.

In another aspect of the present disclosure, there is provided a respiratory system for providing a flow of respiratory gases to a patient, the system comprising: a controller; a flow modulator configured to be controlled by the controller to provide a flow of respiratory gases to a patient; one or more sensors configured to determine pressure of the flow of respiratory gases in the respiratory system to the patient, wherein the controller is configured to: receive an input indicative of pressure of the flow of respiratory gases in the respiratory system from the one or more sensors; compare the pressure against pressure threshold values for corresponding flow rates; control the flow modulator to reduce the flow of respiratory gases in response to the pressure exceeding the pressure threshold values for the corresponding flow rates; and control the flow modulator to modulate the flow of respiratory gases in response to the pressure not exceeding the pressure threshold values for the corresponding flow rates.

The controller of the respiratory system may comprise a microcontroller, a PID (proportional-integral-derivative) controller or a variation of a PID controller where the proportional, integral and derivative elements of the controller can be turned on or off as needed (such as P, PI or I controllers), or some other architecture, configured to operate by an algorithm that is stored in a memory in communication with the controller to direct the operation of controllable components of the respiratory system. The controller thus enables the respiratory system to control one or more components of the respiratory system in response to a change in a pressure and/or flow in the system. The controller may control the flow modulator in response to a change in a pressure in the system. The pressure in the system may be a pressure downstream of the flow modulator. This contrasts with pressure relief valves which typically relieve pressure in the system by venting gases to atmosphere when the system pressure exceeds a pressure threshold. This venting of delivery gases may be considered a source of waste.

The controller of the respiratory system receives an input of a pressure in the system and determines whether the value of the pressure in the system meets or exceeds pressure threshold values for corresponding flow rates, which is indicative of an obstruction (across which there may still be some flow) or blockage (across which there will be no flow). In response the controller controls a component of the respiratory system, which may modulate flow. The controller also restores desired flow to the respiratory system upon subsequent removal of the obstruction or blockage. The controller controlling the flow of respiratory gases to the patient thus reduces wastage of gases, such as Oxygen. It also potentially reduces an undesirable effect on a flow of respiratory gases provided to the patient via another respiratory support system, for example the controller controls the flow of respiratory gases to minimize dilution of anaesthetic agents provided to the patient via an anaesthetic system that is used with the system of the present disclosure. In circumstances as described below where a mask may be used over a cannula, this can also be advantageous by providing better control over the pressure acting on the obstructed/blocked (i.e. collapsed) portion so that a user does not need to apply more force to the mask over the cannula than is required, and to control any residual flow across the collapsed portion associated with the cannula.

In some embodiments, the flow modulator comprises a flow generator, such as a blower, configured to be controlled by the controller to generate a flow of respiratory gases to a patient.

In some embodiments, the flow modulator comprises a proportional valve configured to be controlled by the controller to modulate the flow of respiratory gases.

In some embodiments, the flow modulator further comprises the proportional valve and the flow generator.

The algorithm implemented by the controller controls the flow modulator, such as a blower and/or the proportional valve, to provide pressure/flow control to the respiratory system that supplies gases to a patient during the delivery of respiratory support. Examples of respiratory support are mentioned above, and include nasal high flow, continuous positive airway pressure, and ventilation. The respiratory system may include more than one blower and/or more than one proportional valve.

In some embodiments, the controller is further configured to determine flow rate of the flow of respiratory gases from the input indicative of pressure of the flow of respiratory gases.

In another embodiment, the one or more sensors comprise one or more flow rate sensors configured to sense flow rate of the flow of respiratory gases in the delivery conduit to the patient. The controller is further configured to receive an input indicative of the flow rate of the flow of respiratory gases in the delivery conduit from these one or more flow rate sensors. Preferably, the input indicative of the flow rate is data indicative of the flow rate.

In some embodiments, the pressure of the flow of respiratory gases in the respiratory system is determined from the data indicative of flow rate of the flow of respiratory gases in the respiratory system.

In some embodiments, the one or more sensors are configured to sense pressure of the flow of respiratory gases in the respiratory system downstream of the flow modulator. Preferably, the one or more sensors are located downstream of the flow modulator.

In some embodiments, the one or more sensors comprise one or more pressure sensors configured to sense pressure of the flow of respiratory gases in the system to the patient.

In some embodiments, the one or more sensors comprise the one or more pressure sensors and the one or more flow rate sensors.

In some embodiments, the pressure of the flow of the respiratory gases in the respiratory system is pressure of the flow of respiratory gases downstream of the flow modulator.

In some embodiments, the input indicative of pressure of the flow of respiratory gases in the respiratory system comprises data.

In some embodiments, the respiratory system further comprises a delivery conduit and a patient interface at one end of the delivery conduit, wherein the patient interface is configured to deliver the flow of respiratory gases to the patient.

In some embodiments, the pressure of the flow of the respiratory gases is pressure of the flow of respiratory gases upstream of or at the collapsible portion.

In an example, the patient interface is in fluid communication with a gas supply and the flow of gases is controlled by the flow modulator of the respiratory system. Examples of a gas supply include a pressurised source (such as a gas tank, or a hospital wall supply), a blower, a blender, or a combination thereof. The respiratory system may also include a humidifier for humidifying gases before they are delivered to a patient.

In some embodiments, the patient interface comprises an outlet to be received by a patient nare(s) or mouth; a gases delivery side member extending from a side of the outlet, wherein the gases delivery side member comprises: a lumen for a flow of gases from an inlet of the patient interface to the outlet; and a collapsible portion. For example, the patient interface is a nasal cannula and optionally a non-sealing nasal cannula.

In some embodiments, the collapsible portion is configured to be in a first configuration and collapsible from the first configuration into a second configuration. The flow of respiratory gases through the collapsible portion in the second configuration is at a reduced flow rate compared to when the collapsible portion is in the first configuration. For example, the reduced flow rate is: about 15L/min or less; or about 10L/min or less; or about 10L/min; or about 5L/min to about 10L/min or less than about 5L/min or 0L/min.

In some embodiments, the controller is further configured to control the flow modulator to reduce the flow of respiratory gases to the reduced flow rate in response to the pressure exceeding the pressure threshold values for the corresponding flow rates. For example, the flow of respiratory gases is reduced to the reduced flow rate in a stepped manner. In some embodiments, the controller is configured to control the flow modulator to reduce pressure in the system which in turn reduces the flow rate of respiratory gases (as determined by the change in pressure and resistance to flow within the system). For example, the pressure in the system may be reduced by e.g. slowing operation of the flow modulator in a stepped manner.

In some embodiments, the controller is further configured to control the flow modulator to reduce the flow of respiratory gases continually towards the reduced flow rate. Alternatively/additionally, the controller may be configured to control the flow modulator e.g. by slowing its operation, to reduce the pressure in the system continually towards a pressure target.

In some embodiments, the controller is further configured to control the flow modulator to modulate the flow of respiratory gases to a target flow rate in response to the pressure not exceeding the pressure threshold values for the corresponding flow rates. For example, the target flow rate may be 40 to 70L/min.

In some embodiments, the controller is further configured to control the flow modulator to modulate the flow of respiratory gases continually towards the target flow rate.

In some embodiments, the controller is further configured to control the flow modulator to increase the flow of respiratory gases towards the target flow rate.

In an example, the flow of respiratory gases is increased to the target flow rate in a stepped manner. Alternatively, the controller is configured to control the flow modulator to increase the flow of respiratory gases continually towards the target flow rate.

In some embodiments, the delivery conduit further comprises a delivery circuit and a patient breathing circuit disposed between the delivery circuit and the patient interface, and wherein the patient breathing circuit is connected to the delivery circuit by an outlet connector.

In some embodiments, the delivery conduit further comprises a delivery circuit and a patient breathing circuit disposed between the delivery circuit and the patient interface, and wherein the patient breathing circuit is connected to the delivery circuit by an outlet connector.

In some embodiments, the controller, flow modulator, the one or more sensors and the delivery circuit are housed within a housing, and the outlet connector is mounted to the housing. For example, the housing is a box.

In some embodiments, the one or more sensors comprise an Oxygen (O₂) pressure sensor configured to sense pressure of the flow of O₂ to the patient from an O₂ supply in an O₂ delivery circuit of the respiratory system.

In some embodiments, the one or more sensors comprise an Oxygen (O₂) flow rate sensor configured to sense flow rate of the flow of O₂ to the patient from the O₂ supply.

In some embodiments, the proportional valve is disposed between the O₂ pressure sensor and the O₂ flow rate sensor in the O₂ delivery circuit.

In some embodiments, the one or more sensors comprise an air pressure sensor configured to sense pressure of the flow of air to the patient from ambient air in an air delivery circuit of the respiratory system.

In some embodiments, the blower is disposed after the air pressure sensor in the air delivery circuit.

In some embodiments, the patient breathing circuit is connected to the O₂ delivery circuit and the air delivery circuit and the patient breathing circuit and/or the patient interface further comprises a patient pressure sensor and a patient flow rate sensor.

In some embodiments, the patient breathing circuit is connected to the O₂ delivery circuit and the air delivery circuit and the patient breathing circuit and/or the patient interface further comprises a patient pressure sensor and a patient flow rate sensor.

In some embodiments, the blower is disposed in the patient breathing circuit before the patient pressure sensor and the patient flow rate sensor.

In some embodiments, the pressure threshold values for corresponding flow rates form a pressure limit curve.

In some embodiments, the pressure threshold values for corresponding flow rates form a pressure limit curve. In the embodiment, the controller is further configured to compare the pressure and the flow rate against the pressure limit curve.

In some embodiments, the pressure limit curve is associated with the flow in the respiratory system and/or the delivery conduit being restricted.

In some embodiments, the flow modulator is configured to reduce the flow of respiratory gases by a variable rate of decrease corresponding to the pressure limit curve.

In some embodiments, the pressure limit curve is sigmoidal shaped.

In some embodiments, the sigmoidal shaped pressure limit curve comprises a first pressure region, a second pressure region, and a transition region disposed between the first pressure region and the second pressure region.

In some embodiments, the first pressure region corresponds to the first configuration and the second region corresponds to the second configuration.

In some embodiments, in the first configuration, normal operating pressure of the flow of respiratory gases in the delivery conduit is below the pressure limit curve by a first pressure margin and, in the second configuration, normal operating pressure of the flow of respiratory gases is below the pressure limit curve by a second pressure margin, whereby the first pressure margin is substantially greater than the second pressure margin. The first and the second pressure margin can thus provide a margin for a temporary increase in pressure of the flow of respiratory gases from the normal pressure to not reduce the flow of respiratory gases.

In another embodiment, the first and the second pressure margin provides a margin for a temporary decrease in pressure of the flow of respiratory gases from the normal operating pressure to not increase the flow of respiratory gases.

In some embodiments, the first pressure region is substantially parallel to the second pressure region of the pressure limit curve, and the first pressure region and the second pressure region correspond to a minimum rate of decrease in the flow of respiratory gases.

In some embodiments, the transition region of the pressure limit curve corresponds to a maximum rate of decrease in the flow of respiratory gases.

In some embodiments, the transition region of the pressure limit curve is centred about a transition flow rate.

In some embodiments, the flow modulator is configured to reduce the flow of respiratory gases at the maximum rate of decrease when the pressure and the corresponding flow rate is in the transition region of the pressure limit curve.

In another aspect of the present disclosure, there is provided a respiratory system for providing a flow of respiratory gases to a patient, the system comprising: a controller; a flow modulator configured to be controlled by the controller to provide a flow of respiratory gases to a patient; one or more sensors configured to determine pressure of the flow of respiratory gases in the respiratory system to the patient; and a patient interface in fluid communication with the flow modulator and configured to deliver the flow of respiratory gases to the patient, wherein the patient interface comprises: an inlet to receive the flow of respiratory gases from the flow modulator; an outlet to deliver the flow of respiratory gases to an airway of the patient; and a gases conduit comprising a collapsible portion configured to switch between a first configuration and a second configuration, wherein the pressure of the flow of respiratory gases in the respiratory system when the collapsible portion is in the second configuration is greater than the pressure of the flow of the respiratory gases in the respiratory system when the collapsible portion is in the first configuration, wherein the controller is configured to: receive an input indicative of pressure of the flow of respiratory gases in the respiratory system from the one or more sensors; compare the pressure against a pressure threshold; control the flow modulator to provide a first modulation of the flow of respiratory gases in response to the pressure meeting or exceeding the pressure threshold; and control the flow modulator to provide a second modulation of the flow of respiratory gases in response to the pressure not meeting or exceeding the pressure threshold.

In some embodiments, the second modulation is different from the first modulation.

In some embodiments, the first modulation comprises reducing or maintaining the flow of the respiratory gases.

In some embodiments, the first modulation comprises reducing the flow of the respiratory gases in response to the pressure exceeding the pressure threshold.

In some embodiments, the first modulation comprises reducing or maintaining the flow of the respiratory gases in response to the pressure meeting the pressure threshold.

In some embodiments, the second modulation comprises increasing the flow of respiratory gases.

In some embodiments, the second modulation comprises modulating the flow of respiratory gases to a target flow rate.

In some embodiments, the one or more sensors are located downstream of the flow modulator.

In some embodiments, the respiratory system further comprises a delivery conduit configured to deliver the flow of respiratory gases to the patient interface from the flow modulator.

In some embodiments, the delivery conduit further comprises a delivery circuit and a patient breathing circuit disposed between the delivery circuit and the patient interface, and wherein the patient breathing circuit is connected to the delivery circuit by an outlet connector.

In some embodiments, the controller, flow modulator, the one or more sensors and the delivery circuit are housed within a housing, and the outlet connector is mounted to the housing.

In some embodiments, the flow modulator comprises a flow generator configured to be controlled by the controller to generate a flow of respiratory gases to a patient.

In some embodiments, the flow modulator comprises a proportional valve configured to be controlled by the controller to modulate the flow of respiratory gases.

In some embodiments, the flow modulator further comprises the proportional valve and the flow generator.

In some embodiments, the flow generator comprises a blower configured to be controlled by the controller to generate the flow of respiratory gases.

In some embodiments, the one or more sensors comprise an Oxygen (O₂) pressure sensor configured to sense pressure of the flow of O₂ to the patient from an O₂ supply in an O₂ delivery circuit of the respiratory system.

In some embodiments, the one or more sensors comprise an Oxygen (O₂) flow rate sensor configured to sense flow rate of the flow of O₂ to the patient from the O₂ supply.

In some embodiments, the proportional valve is disposed between the O₂ pressure sensor and the O₂ flow rate sensor in the O₂ delivery circuit.

In some embodiments, the one or more sensors comprise an air pressure sensor configured to sense pressure of the flow of air to the patient from ambient air in an air delivery circuit of the respiratory system.

In some embodiments, the blower is disposed after the air pressure sensor in the air delivery circuit.

In some embodiments, the patient breathing circuit is connected to the O₂ delivery circuit and the air delivery circuit and the patient breathing circuit and/or the patient interface further comprises a patient pressure sensor and a patient flow rate sensor.

In some embodiments, the blower is disposed in the patient breathing circuit before the patient pressure sensor and the patient flow rate sensor.

In some embodiments, the one or more sensors comprise one or more pressure sensors configured to sense pressure of the flow of respiratory gases in the system to the patient. The one or more pressure sensors are configured to sense pressure of the flow of respiratory gases in the respiratory system downstream of the flow modulator.

In some embodiments, the input indicative of pressure of the flow of respiratory gases in the respiratory system comprises data.

In some embodiments, the pressure of the flow of the respiratory gases in the respiratory system is pressure of the flow of respiratory gases downstream of the flow modulator.

In some embodiments, the pressure of the flow of the respiratory gases is pressure of the flow of respiratory gases upstream of or at the collapsible portion.

In another aspect of the present disclosure, there is provided a method of operating a respiratory system for providing a flow of respiratory gases to a patient, the method comprising: providing a flow of respiratory gases to a patient with the respiratory system; determining pressure of the flow of respiratory gases to the patient in the respiratory system using one or more sensors configured to determine pressure of the flow of respiratory gases in the respiratory system; receiving an input indicative of pressure of the flow of respiratory gases in the system from the one or more sensors; comparing the pressure against pressure threshold values for corresponding flow rates; controlling the flow modulator to reduce the flow of respiratory gases in response to the pressure exceeding the pressure threshold values for the corresponding flow rates; and controlling the flow modulator to modulate the flow of respiratory gases in response to the pressure not exceeding the pressure threshold values for the corresponding flow rates.

In another aspect of the present disclosure, there is provided a method of operating a respiratory system as described above, the method comprising: providing a flow of respiratory gases to a patient with the respiratory system; determining pressure of the flow of respiratory gases to the patient in the respiratory system using one or more sensors configured to determine pressure of the flow of respiratory gases in the respiratory system; receiving an input indicative of pressure of the flow of respiratory gases in the system from the one or more sensors; comparing the pressure against pressure threshold values for corresponding flow rates; controlling the flow modulator to reduce the flow of respiratory gases in response to the pressure exceeding the pressure threshold values for the corresponding flow rates; and controlling the flow modulator to modulate the flow of respiratory gases in response to the pressure not exceeding the pressure threshold values for the corresponding flow rates.

### Brief Description of Drawings

Embodiments of the disclosure will now be described in greater detail with reference to the following Figures:
Figure 1 is a schematic diagram of an example of a respiratory system for providing respiratory gases to a patient;
Figure 2a and 2b show a patient wearing a first patient interface (Figure 2a) and a first patient interface with a second patient interface (Figure 2b) for use with a respiratory system for providing respiratory gases to a patient according to an embodiment of the present disclosure;
Figures 3a and 3b provide schematic illustrations of the first and second configurations of a collapsible portion of the patient interface; Figure 3a shows the first configuration and Figure 3b shows the second configuration;
Figure 4 is a schematic diagram of a respiratory system for providing respiratory gases to a patient according to an embodiment of the present disclosure;
Figure 5 is a schematic diagram of a respiratory system for providing respiratory gases to a patient according to an embodiment of the present disclosure;
Figure 6 is a schematic diagram of a respiratory system for providing respiratory gases to a patient according to an embodiment of the present disclosure;
Figure 7 is a schematic diagram of a respiratory system for providing respiratory gases to a patient according to an embodiment of the present disclosure;
Figure 8 is a schematic diagram of a respiratory system for providing respiratory gases to a patient according to an embodiment of the present disclosure;
Figure 9 is a graph illustrating a curve that represents a relationship between flow and pressure, used in controlling a flow of respiratory gases according to an embodiment of the present disclosure;
Figure 10 is a graph illustrating a curve that represents a relationship between flow and pressure, used in controlling a flow of respiratory gases according to an embodiment of the present disclosure;
Figure 11 is a graph illustrating another curve that represents a relationship between flow and pressure, used in controlling a flow of respiratory gases according to an embodiment of the present disclosure;
Figure 12 is a graph illustrating that at zero flow the pressure curve can have a nominal pressure limit (curve R) or a zero pressure limit (curve S).
Figure 13 is a graph illustrating a curve that represents a relationship between flow and pressure offset, used in controlling a flow of respiratory gases according to an embodiment of the present disclosure;
Figure 14 is a graph illustrating the changing relationship between pressure and flow rate as an increasing resistance to flow is experienced by the system.
Figure 15 is a state diagram of the states of operation of a respiratory system according to an embodiment of the present disclosure;
Figure 16 is a flow chart of operation of a respiratory system according to an embodiment of the present disclosure; and
Figure 17 is a flow chart of a method of operating a respiratory system according to an embodiment of the present disclosure.

### Detailed Description

Throughout the figures and specification, similar reference numerals may be used to designate the same or similar components, and redundant descriptions thereof may be omitted.

As mentioned, respiratory systems provide gas for delivery to a patient. Respiratory systems may take a number of forms, such as continuous positive airway pressure systems (CPAP) and high flow respiratory gas systems (e.g. for use in high flow therapy and anaesthesia procedures).

In this specification, "high flow" means, without limitation, any gas flow with a flow rate that is higher than usual/normal, such as higher than the normal inspiration flow rate of a healthy patient. Alternatively, or additionally, it can be higher than some other threshold flow rate that is relevant to the context - for example, where providing a gas flow to a patient at a flow rate to meet inspiratory demand, that flow rate might be deemed "high flow" as it is higher than a nominal flow rate that might have otherwise been provided. "High flow" is therefore context dependent, and what constitutes "high flow" depends on many factors such as the health state of the patient, type of procedure/therapy/support being provided, the nature of the patient (big, small, adult, child) and the like. Those skilled in the art would appreciate from context what constitutes "high flow". It is a magnitude of flow rate that is over and above a flow rate that might otherwise be provided.

But, without limitation, some indicative values of high flow can be as follows.

In some configurations, delivery of gases to a patient at a flow rate of greater than or equal to about 5 or 10 litres per minute (5 or 10 LPM or L/min).

In some configurations, delivery of gases to a patient at a flow rate of about 5 or 10 LPM to about 150 LPM, or about 15 LPM to about 95 LPM, or about 20 LPM to about 90 LPM, or about 25 LPM to about 85 LPM, or about 30 LPM to about 80 LPM, or about 35 LPM to about 75 LPM, or about 40 LPM to about 70 LPM, or about 45 LPM to about 65 LPM, or about 50 LPM to about 60 LPM. For example, according to various embodiments and configurations described herein, a flow rate of gases supplied or provided to an interface via a system or from a flow source or flow modulator, may comprise, but is not limited to, flows of at least about 5, 10, 20, 30, 40, 50, 60, 70, 80, 90, 100, 110, 120, 130, 140, 150 LPM, or more, and useful ranges may be selected to be any of these values (for example, about 20 LPM to about 90 LPM, about 40 LPM to about 70 LPM, about 40 LPM to about 80 LPM, about 50 LPM to about 80 LPM, about 60 LPM to about 80 LPM, about 70 LPM to about 100 LPM, about 70 LPM to about 80 LPM).

In "high flow" the gas delivered will be chosen depending on for example the intended use of a therapy. Gases delivered may comprise a percentage of oxygen. In some configurations, the percentage of oxygen in the gases delivered may be about 15% to about 100%, 20% to about 100%, or about 30% to about 100%, or about 40% to about 100%, or about 50% to about 100%, or about 60% to about 100%, or about 70% to about 100%, or about 80% to about 100%, or about 90% to about 100%, or about 100%, or 100%.

In some embodiments, gases delivered may comprise a percentage of carbon dioxide. In some configurations, the percentage of carbon dioxide in the gases delivered may be more than 0%, about 0.3% to about 100%, about 1% to about 100%, about 5% to about 100%, about 10% to about 100%, about 20% to about 100%, or about 30% to about 100%, or about 40% to about 100%, or about 50% to about 100%, or about 60% to about 100%, or about 70% to about 100%, or about 80% to about 100%, or about 90% to about 100%, or about 100%, or 100%.

Flow rates for "High flow" for premature/infants/paediatrics (with body mass in the range of about 1 to about 30 kg) can be different. The flow rate can be set to 0.4-8 L/min/kg with a minimum of about 0.5 L/min and a maximum of about 70 L/min. For patients under 2 kg maximum flow may be set to 8 L/min.

High flow has been found effective in meeting or exceeding the patient's normal inspiratory flow, to increase oxygenation of the patient and/or reduce the work of breathing. Additionally, high flow therapy may generate a flushing effect in the nasopharynx such that the anatomical dead space of the upper airways is flushed by the high incoming gas flows. This creates a reservoir of fresh gas available for each and every breath, while minimising re-breathing of carbon dioxide, nitrogen, etc.

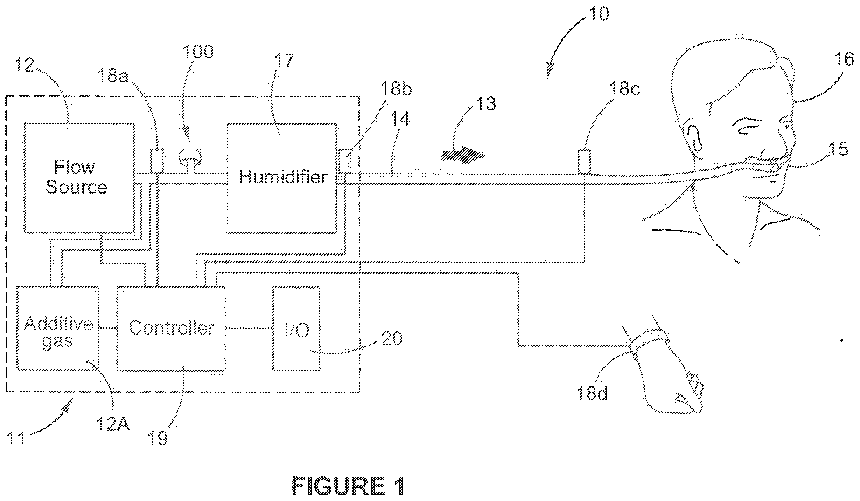

By example, a high flow respiratory system 10 is described with reference to Figure 1. High flow may be used as a means to promote gas exchange and/or respiratory support through the delivery of oxygen and/or other gases, and through the removal of CO₂ from the patient's airways. High flow may be particularly useful prior to, during or after a medical and/or anaesthetic procedure.

When used prior to a medical procedure, high gas flow can pre-load the patient with oxygen so that their blood oxygen saturation level and volume of oxygen in the lungs is higher to provide an oxygen buffer while the patient is in an apnoeic phase during the medical procedure.

A continuous supply of oxygen is important to sustain healthy respiratory function during medical procedures (such as during anaesthesia) where respiratory function might be compromised (e.g. diminishes or stops). When this supply is compromised, hypoxia, hypoxaemia, and/or hypercapnia can occur. During medical procedures such as anaesthesia and/or sedation where the patient is unconscious or may become unconscious, the patient is monitored to detect when this happens. If oxygen supply and/or CO₂ removal is compromised, the clinician stops the medical procedure and facilitates oxygen supply and/or CO₂ removal. This can be achieved for example by manually ventilating the patient for example by bag mask ventilation, or by providing a high flow of gases to the patient's airway using a high flow respiratory system. Further, it will be appreciated that a mask that is used for sedation/ventilation (not necessarily limited to a bag mask) may also be used for pre-oxygenation and also for monitoring patient parameters such as end tidal CO₂, etc.

Further advantages of high gas flow can include that the high gas flow increases pressure in the airways of the patient, thereby providing pressure support that opens airways, the trachea, lungs/alveolar and bronchioles. The opening of these structures enhances oxygenation, and to some extent assists in removal of CO₂.

When humidified, the high gas flow can also prevent airways from drying out, mitigating mucociliary damage, and reducing risk of laryngospasms and risks associated with airway drying such as nose bleeding, aspiration (as a result of nose bleeding), and airway obstruction, swelling and bleeding. Another advantage of high gas flow is that the flow can clear smoke created during surgery in the air passages. For example, smoke can be created by lasers and/or cauterizing devices.

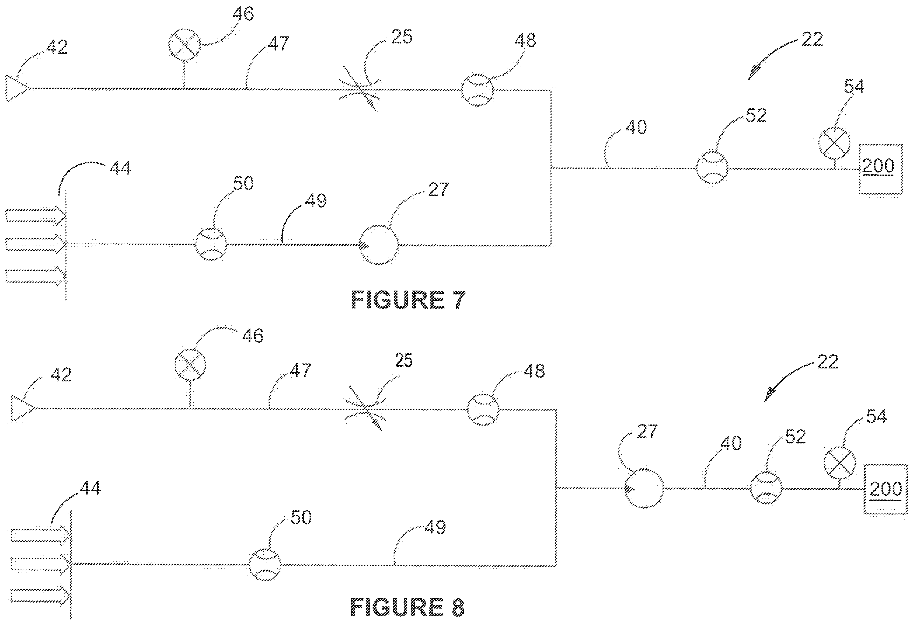

With reference to Figure 1, the system 10 may comprise an integrated or separate component-based arrangement, generally shown in the dotted box 11 in Figure 1. In some configurations, the system 10 could comprise a modular arrangement of components. The system 10 may include a flow source 12, such as an in-wall source of oxygen, an oxygen tank, a blower, a flow therapy apparatus, or any other source of oxygen or other gas or combination thereof. In some embodiments, the flow source 12 comprises a flow modulator and in some embodiments, the flow modulator comprises a flow generator such as a blower, bellow, and/or pistons. In some embodiments, the flow modulator comprises a flow generator and a proportional valve which may function to control oxygen concentration in a flow of blended gas such as air (preferably filtered air) and oxygen which is delivered to the patient. In some embodiments, the flow modulator comprises a proportional valve, and in such embodiments, the flow modulator may not comprise a flow generator. An example of a system comprising a flow generator and proportional valve in this context is described in relation to Figures 7 and 8. In other embodiments, the flow source 12 need not comprise a flow generator and in such embodiments, the flow source 12 may comprise an in-wall gas source and/or a blended gas or other gas supply. In some embodiments, the flow source 12 may comprise a compressed gas source (e.g. an in-wall gas source, an oxygen tank supply, etc.) and a blower.

In some embodiments, the flow source 12 comprises or is part of an anaesthesia machine. The system 10 may also comprise an additive gas source 12A, comprising one or more other gases that can be combined with gases from the flow source 12. The flow source 12 can provide a flow of gas 13 that can be delivered to a patient 16 via a delivery conduit 14, and patient interface 15 (such as a nasal cannula). The flow of gas 13 may deliver a high flow to the patient, in the context described in the foregoing. A controller 19 controls the flow source 12 and additive gas source 12A through valves or the like to control flow and other characteristics such as any one or more of flow rate, pressure, composition, concentration, volume of the flow of gas 13. A humidifier 17 is also optionally provided, which can humidify the gas and/or control the temperature of the gas, for example under the control of the controller 19. One or more sensors 18a, 18b, 18c, 18d, such as flow, oxygen, pressure, humidity, temperature or other sensors can be placed throughout the system and/or at, on or near the patient 16. The sensors can include a pulse oximeter 18d on the patient for determining the oxygen concentration in the blood.

The controller 19 may be operatively coupled with one or more components of system 10 by various means including wired or wireless coupling. For example, controller 19 may be operatively coupled with one or more of the flow source 12, the additive gas source 12A, humidifier 17 and sensors 18a-18d and input/output (I/O) interface 20. By way of example, the controller 19 may be provided on or in a high flow apparatus, a separate component and/or incorporated into or utilised with another device such as an anaesthesia machine or a ventilator, or it may comprise part of system 10 and communicate with one or more separate controllers controlling operation of separate components used with system 10 for the provision of respiratory support to the patient. The controller may comprise a microcontroller, a PID (proportional-integral-derivative) controller or a variation of a PID controller where the proportional, integral and derivative elements of the controller can be turned on or off as needed (such as P, PI or I controllers), or some other architecture, configured to operate by an algorithm that is stored in a memory in communication with the controller to direct the operation of controllable components of the respiratory system. The controller 19 may thus control the flow source 12 and other components of or used with system 10 to provide the delivered flow of gas to the patient with certain characteristics such as a desired flow rate, pressure, composition (where more than one gas is being provided), volume and/or other parameters based on feedback from one or more sensors 18a-18d. The controller 19 can also control any other suitable parameters of the flow source to meet oxygenation, airway pressure and/or flow requirements of the patient and/or system pressure and/or flow requirements of the system (for example pre-determined or set by a user through interface 20). The controller 19 can also control the humidifier 17 and this control may be based on feedback from one or more of the sensors 18a-18d. Using input from the sensors, the controller may determine operational changes required to meet oxygenation requirements and alter control parameters of the flow source 12 and/or humidifier 17 and/or other additive gas source 12A and/or other components of the system as required.

An input/output (I/O) interface 20 (such as a display and/or input device) may be provided. The interface 20 enables information and inputs (such as the required patient respiratory support parameters) to be received from a user (e.g. clinician or patient) that can be used for determining oxygenation, pressure, flow requirements and/or other system settings used in the control of one or more of the flow source 12, additive gas source 12A and other components of the system 10, to achieve a flow of gas 13 with the characteristics necessary to provide the required respiratory support. In some embodiments, the system may be without a controller and/or I/O interface. A medical professional such as a nurse or technician may provide the necessary control function.

As noted above, the high gas flow (optionally humidified) may be delivered to the patient 16 via a delivery conduit 14 and the patient interface 15 or 'interface', such as a cannula, mask, nasal interface, oral device or combination thereof. In some embodiments, the high gas flow (optionally humidified) may be delivered to the patient 16 for surgical uses, e.g. surgical insufflation. In these embodiments, the 'interface' could be a surgical cannula, trocar, or other suitable interface. The patient interface may seal, substantially seal, partially seal, be non-sealing, substantially non-sealing, or partially non-sealing with a patient's airways. A nasal interface as used herein is a device such as a cannula, a nasal mask, nasal pillows, or other type of nasal device or combinations thereof configured to direct a flow of gas into one or both nares of the patient.

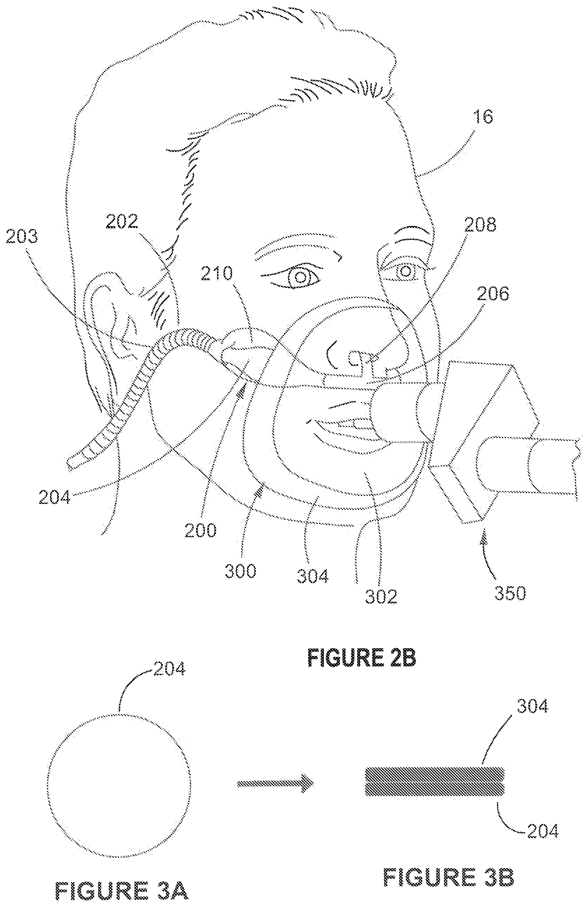

A nasal interface can also be used in combination with a face mask 300, as shown in Figure 2b, or oral device (such as a tube inserted into the mouth) and/or a mask or oral device (such as a tube inserted into the mouth) that can be detached and/or attached to the nasal interface.

A nasal cannula is a nasal interface that may include one or more prongs that are configured to be inserted into a patient's nasal passages. A mask refers to an interface that covers a patient's nasal passages and/or mouth and can also include devices in which portions of the mask that cover the patient's mouth are removable. A mask also refers to a nasal interface that includes nasal pillows that create a substantial seal with the patient's nostrils.

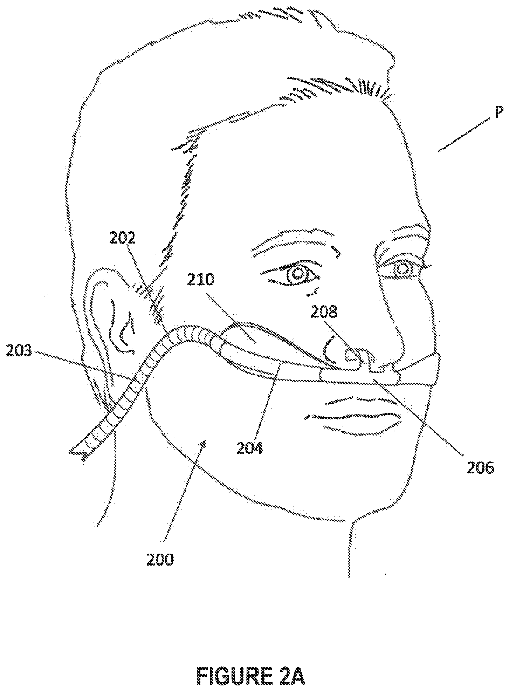

Figures 2a and 2b show examples of a patient 16 wearing a patient interface 200, for example the nasal cannula 15 of the respiratory system 10 of Figure 1, with a collapsible breathing conduit portion. The patient depicted is an adult. However, the patient may be an infant, child or adolescent.

The patient interface 200 comprises a first gas (delivery) conduit 202. The first gas conduit 202 is adapted to receive gases from the respiratory system 10 of Figure 1 (for example, via the conduit 14 shown in Figure 1) and direct the gases to the patient 16. The first gas conduit 202 may comprise a reinforcement element 203 adapted to strengthen and/or add rigidity to the first gas conduit to prevent deformation or collapse of the first gas conduit 202 arising due to the application of forces against the first gas conduit 202. The reinforcement element 203 may include a number of structures, including but not limited to plastic or metallic reinforcing beads that lie in or on the wall of the first conduit lumen 202.

The patient interface 200 may comprise a gases delivery side arm in fluid communication with the first gas conduit 202. The first gas conduit 202 is in pneumatic communication with a flow manifold 206 which is provided at an end of the gases delivery side arm. The flow manifold 206 receives gases from the first gas conduit 202 and provides passage to one or more nasal delivery elements 208 (e.g. nasal prongs) extending from the manifold. The one or more nasal delivery elements 208 extend outwardly from the flow manifold 206. The one or more nasal delivery elements 208 are adapted to be non-sealingly positioned in one or more nares of the patient 16. First patient interface 200 is accordingly a non-sealing patient interface. As shown, the patient interface 200 comprises two nasal prongs 208 adapted to be positioned with one in each of the patient's nares. Each nasal prong 208 may be shaped or angled such that it extends inwardly towards a septum of the patient's nose. Alternatively, the first patient interface 200 may be a sealing nasal interface.

In the embodiment shown in Figures 2a and 2b, the flow manifold 206 receives flow from one lateral side of the flow manifold 206 (e.g. with respect to an imaginary vertical plane bisecting the face of the patient P) and provides a passage for flow through to the manifold to each of the nasal prongs 208. In some embodiments a conduit may extend from a single side of the manifold, for example from the left-hand side or from the right-hand side of the manifold. In some situations, providing the conduit on the left-hand side of the patient interface may be preferred for access by a clinician, for example for intubation. Alternatively, a conduit extending from the right-hand side may be preferred, for example in procedures such as endoscopies where the patient is typically lying on his or her left-hand side. In other configurations, the patient interface 200 may comprise greater (for example, three or four) or fewer (for example, one) nasal delivery elements 208. In other configurations, each of the nasal delivery elements 208 can have different properties. For example, one of a pair of nasal delivery elements 208 can be relatively long and the other nasal delivery elements 208 can be relatively short.

In some configurations, the flow manifold 206 may be configured to receive flow from two lateral sides of the flow manifold 206 (e.g. from a 'left' and 'right' of the flow manifold 206 instead of just the patient's right-hand side of the flow manifold 206 as seen in Figures 2a and 2b). In some such configurations, multiple gas conduits may be used to provide for pneumatic communication between the flow manifold 206 and the respiratory system 10. For example, the patient interface may comprise dual conduits, the first gas conduit 202 extending from a first side of the interface (in the illustrated example the right-hand side of the patient) and a second gas conduit (not shown) extending from a second opposite side of the interface. In some configurations, the flow manifold 206 may be configured to receive flow from a nonlateral side of the flow manifold 206 (e.g. from a 'bottom' or 'top' of the flow manifold 206 or both) or from a front face of the flow manifold 206, opposite the patient 16.

The patient interface 200 may further comprise mounts and/or supports, e.g., cheek supports 210, for attaching and/or supporting the gas conduit 202 or conduits on the patient's face. Alternatively, or additionally, the patient interface 200 may be held in place via one or more head straps or headgear (not shown).

The first gas conduit 202 of the patient interface 200 comprises a first portion 204 configured to transition from a first configuration in which a first level of gases is able to pass through the first portion 204 to a second configuration in which a second level of gases is able to pass through the first portion 204.

Figure 2b shows the patient 16 wearing the patient interface 200 comprising two nasal prongs 208 simultaneously underneath a face mask assembly 300 (a second patient interface). In this arrangement, face mask assembly 300 is placed upon the patient interface 200 which is worn by patient 16. Figure 2b schematically shows the face mask assembly 300 as a transparent structure in order to illustrate the patient interface 200 under it. The first patient interface 200 may be used with a first respiratory support system 10 and the face mask assembly (second patient interface) 300 may be used together with a second respiratory support system (not shown). In some configurations, the first and second respiratory support systems are the same system and/or the first and second respiratory support systems comprise a common flow source despite the modes of respiratory support being provided by the first and second respiratory support systems being different. In other configurations, the first and second respiratory support systems are separate systems.

The configuration shown in Figure 2b may be beneficial in the provision of selective delivery of separate therapies or modes of support to a patient using different patient interfaces, and/or in stopping or ceasing the delivery of a therapy from an interface and/or allowing gases provided by an interface to be sampled. For example, the configuration may find particular application in emergency resuscitation, around intubation of a patient receiving high flow therapy, ear, nose, and throat (ENT) surgery, in assisting with conditioning of a patient in a pre-operative state prior to administration of anaesthetics, and during post-extubation and recovery.

The face mask assembly 300 may be used as or with a second respiratory support system and/or to deliver one or more substances other than a substance delivered by the cannula 200. For example, for delivery of anaesthetic agents and/or oxygen, to the patient, or for delivery of the same substance as the first patient interface 200 but at different flow and/or pressure levels. Alternatively, the face mask assembly 300 may be used to reduce or stop the delivery of therapy from a first respiratory support system through the first patient interface 200. In some embodiments, the face mask assembly 300 may also be adapted to measure respiratory gases, for example exhaled carbon dioxide from the patient, the measurements of which may otherwise be affected by flow from the patient interface 200 of the first respiratory support system.

The configuration shown in Figure 2b allows for the alternation between the two different respiratory support systems. Additionally, this configuration may allow the first patient interface 200 to be left on the patient throughout a medical procedure and/or into recovery (whether or not the patient continues to receive therapy through the patient interface 200 throughout the procedure) without interfering with other clinical practices.

In the embodiment shown, the face mask assembly 300 comprises a fullface mask 302 configured to cover both the patient's nose and mouth. In other configurations, the face mask assembly 300 may comprise a nasal mask which is placed over the patient interface 200 to cover only the patient's nasal region.

As shown, the face mask 302 comprises a seal region 304 adapted to seal against the patient's face. The face mask assembly 300 is connected to a second gas source, for example via a filter element 350, which supplies the one or more other gases to the patient via the face mask. In some configurations, the second gas source is different from the source supplying gas (for example a supplementary gas source or flow generator) to the patient interface 200. In some configurations, the second gas source is the same as the source supplying gas to the patient interface 200.

In some embodiments, the face mask assembly 300 is connected to a second flow source that is or comprises a separate gas source or a separate respiratory support system configured to provide respiratory support separate from any flow source, or respiratory support system delivering a flow of gas to the first patient interface 200. For example, the separate respiratory support system can be a ventilator or a CPAP or a high flow therapy device or a manual resuscitator (for example a hand-held face mask with bag). Alternatively or additionally, the face mask assembly 300 may be connected to a device for measuring a characteristic of respiratory gases.

Alternatively, the separate respiratory support system may be or comprise an anaesthetic device and/or the second gas source may comprise an anaesthetic gas, or air, or oxygen, or a combination of gases, for delivery via the face mask 302.

The configuration shown in Figure 2b allows for the delivery of gas from multiple sources via at least two different respiratory support modes, and further allows a doctor, clinician or medical professional to quickly and easily change the type of respiratory support mode.

In one particular application, a patient undergoing an anaesthetic procedure may undergo pre-oxygenation by delivering a high flow of oxygen or humidified gases or mixture of both, for example via a nasal cannula, when the patient is still spontaneously breathing and before the administration of anaesthetic agents. Pre-oxygenation increases the patient's oxygen reservoir prior to the anaesthetic procedure. The term "anaesthetic procedure" may refer, without limitation, to general anaesthesia, procedural sedation and regional/local anaesthesia. In some circumstances, anaesthetists managing the anaesthetic procedure of a patient may want to switch between delivery of gas flow from one patient interface (for example a nasal cannula 200) and delivery of gas flow from another patient interface, such as via a face mask 300.

Anaesthetists also use a mask with a bag to oxygenate a patient, and in some instances find it more beneficial to use a bag mask if a patient's vital signs begin to drop for example to deliver more pressure to support the patient's airways, or to have greater manual control over the variation in delivered pressure. In some situations, a medical professional may wish to switch between different respiratory systems or support modes. In a first mode, respiratory support may be provided by first respiratory support system (for example via the patient interface 200) and in a second mode respiratory support may be provided by a second respiratory support system (for example via the face mask assembly 300), with the support from the first system reduced or stopped. For example, it may be desirable to stop high flow from a first patient interface 200 when delivering anaesthetic agents through the face mask assembly 300 because a high flow from interface 200 may modify the expected behaviour of the anaesthetic circuit provided by the face mask 300 (which is typically a sealed circuit) and may dilute anaesthetic agents delivered by face mask assembly 300. Thus, it may be advantageous to be able to stop the additional flow from the first respiratory system or substantially reduce it.

In some configurations, the switching between two respiratory support modes or subsystems may be facilitated by a structure of the first gas conduit 202, which has first portion 204 configured to transition between a first configuration in which a first level of gases is able to pass through the first portion 204 and a second configuration in which a second level of gases is able to pass through the first portion 204.

In some configurations, the first portion 204 is configured to be more collapsible or otherwise better adapted for changing the flow of gas through the first portion 204 (to reduce the flow of gas through the conduit and to the patient) than other portions of the conduit 202, and/or allowing a seal of a mask to seal over the top of the conduit. In other configurations the entire conduit 202 may be configured to be collapsible or otherwise better adapted for changing the flow of gas through conduit 202. In some configurations a vent arrangement may be provided upstream of a collapsible portion, to vent gases from the conduit upstream of the collapsible portion to atmosphere.

In some embodiments, the first configuration is a fully or substantially open condition, and the second configuration is a fully or substantially closed condition. That is, the conduit 202 is configured to be more collapsible, deformable or otherwise adapted to fully or substantially close off the flow at the first portion 204 than at other portions of the conduit 202, when in the second configuration. It will be understood that there may be one or more intermediate conditions between the first and second configurations, where these one or more intermediate conditions may be less open (or more closed) than the fully or substantially open condition (first configuration) but more open (or less closed) than the fully or substantially closed condition (second configuration).

Figures 3a and 3b provide schematic illustrations of the first and second configurations wherein Figure 3a shows the first configuration (substantially open) and Figure 3b shows the first portion 204 in the second configuration (substantially closed) by application of the seal 304 of face mask 302 over the first portion 204. In some embodiments, the first portion 204 (i.e. the more collapsible or deformable section) of the first gas conduit 202 should be of a length that is greater than a width of a section of a seal 304 of the face mask 302 that bears over the first portion 204 of the first gas conduit 202. This ensures the seal of the face mask 302 does not bear over a non-collapsible section of the first gas conduit 202. For example, the first portion 204 may extend from a distance of 35mm or less from the centre of a user's nose to at least 50mm from the centre of a user's nose. The first portion 204 may have a length of at least about 5mm, about 1mm to about 30mm in length, or about 5mm to about 15mm in length, or about 10mm in length. In some embodiments the length of the first portion may be at least 1mm, 2mm, 3mm, 4mm, 5mm, 6mm, 7mm, 8mm, 9mm, 10mm, 11mm, 12mm, 13mm, 14mm, 15mm, 20mm, 25mm, 30mm, 35mm, 40mm, 45mm, 50mm or greater.

The first portion 204 may progress between the first and second configurations based on a relative level of force applied to a wall of the first portion 204. For example, as shown in Figures 2 and 3, the force may be applied by the seal 304 of face mask 302. In this example, first portion 204 is configured to be positioned under the seal 304 of the face mask 302.

Alternatively, the force may be applied to first portion 204 by other means, e.g., clamps (not shown), or alternatively a medical practitioner may compress the conduit by pressing on the conduit wall with a finger or thumb.

In some embodiments, the seal of the face mask acting on the first portion 204 of the gas conduit 202 causes the first portion 204 to form a seal or at least a partial seal between the nasal outlets of the first patient interface 200 and the flow source 12. Additionally, the seal of the face mask forms a seal or at least a partial seal over the first portion 204 of the gas conduit 202.

Switching between respiratory support therapies may therefore be achieved simply by applying a mask to the patient's face so that the seal of the mask collapses (partially or completely) the first portion 204 of the gas conduit 202 supplying the first interface 200 to reduce or stop the therapy supplied by the first interface 200. This also provides a seal between the face mask 300 and the external surface of the first portion 204 of the conduit 202 such that respiratory support or therapy can be provided by the face mask 300 where the respiratory support or therapy provided by the first patient interface 200 can be reduced or shut off. The patient interface 200 with a collapsible conduit portion 204 allows a user, e.g. an anaesthetist or a nurse or a clinician, to use a face mask assembly 300 over the patient interface 200 to select and control delivery of gases from multiple respiratory support systems to provide different therapies or modes of support. The first patient interface 200 may be structured to function in a manner that prevents the delivery of high flow and other respiratory therapy or anaesthetic agents through the patient interface 200 when the first portion 204 is in a second configuration. In some embodiments removal of the face mask assembly 300 from the patient's face allows the first portion 204 to return to its first configuration so that respiratory support or therapy supplied by the first patient interface 200 can recommence or return to operating in the conditions present prior to the change in configuration.

Embodiments of the present disclosure provide for improved control of a flow of respiratory gases provided to a patient. In some embodiments, the respiratory support system includes a pressure relief valve or device. The system 10 may include such a pressure relief or regulating device, or pressure limiting device 100 (e.g. a pressure relief valve or PRV). This pressure limiting device 100 may be a valve having features described in WO2018/033863, the entirety of which is incorporated by reference herein. In some embodiments, the respiratory system does not include, or excludes a pressure relief valve or device. In some embodiments, the respiratory system does not include, or excludes a flow compensated pressure relief valve or device, for example, a flow compensated pressure relief valve having features as described in WO2018/033863.

Pressure relief and pressure control are particularly desirable for use in a respiratory support system such as a high flow system 10 comprising a non-sealing patient interface (such as a nasal cannula in first patient interface 200), to provide an upper limit on pressures that may be generated downstream from the flow source 12 which in turn can impact patient airway pressure (also referred to as patient pressure). Importantly, the upper pressure limit may be configured to provide a safety threshold, to ensure patient pressure safety, and/or to prevent damage to tubes, fluid connections, or other components in the system 10 due to over-pressure. Similarly, a pressure relief or regulating device 100 may be used in a sealed system, such as CPAP (continuous positive airway pressure), BiPAP (bilevel positive airway pressure) and/or Bubble CPAP systems to regulate the pressure provided to the patient.

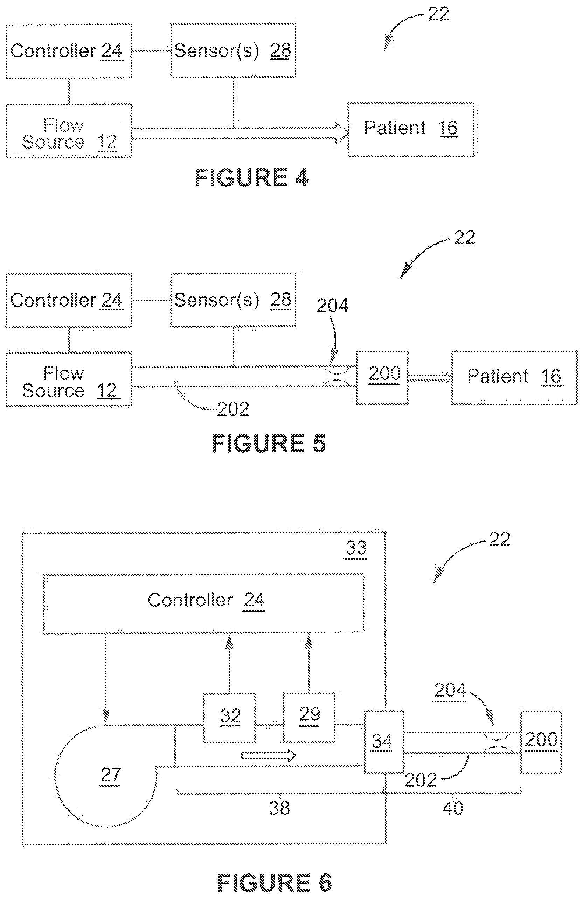

A respiratory system 22 for providing a flow of respiratory gases to a patient is shown in Figures 4 to 8 according to different embodiments of the present disclosure.

Figure 4 shows a respiratory system 22 comprising a controller 24 and a flow source 12 which may comprise a flow modulator configured to be controlled by the controller 24 to modulate a flow of respiratory gases to a patient 16. The controller may be configured to receive an input of a flow rate and a pressure of the flow of respiratory gases in the system, and be configured to control the flow source to provide the flow of respiratory gases at a target flow rate to a patient and control the flow modulator to modulate the flow of respiratory gases to a target pressure when the pressure of the flow of respiratory gases in the system meets or exceeds a pressure threshold value corresponding to that target flow rate. In some embodiments, the target pressure comprises the pressure threshold value corresponding to the target flow rate or a pressure threshold value corresponding to a flow rate less than the target flow rate. The pressure threshold value corresponding to the target flow rate may be determined by the controller e.g. using a function stored in a memory component of or operatively coupled with the controller as described elsewhere herein. A target pressure or pressure threshold may be defined as an absolute pressure value or a gauge pressure value.

As mentioned, the flow source 12 may comprise a flow generator, such as a blower, configured to be controlled by the controller 24 to generate the flow of respiratory gases. Alternatively, or additionally, the flow source 12 may comprise a proportional valve configured to be controlled by the controller 24 to modulate the flow of respiratory gases from a gas source, or the flow source 12 may comprise a combination of a flow generator and a proportional valve.

Typically, the system 22 further comprises one or more sensors 28 configured to determine characteristics of the respiratory system, for example characteristics of the flow of respiratory gases in the respiratory system and/or being provided to the patient. The system 10 can use ultrasonic transducer(s), flow rate sensor(s) such as thermistor flow sensor(s), pressure sensor(s), temperature sensor(s), humidity sensor(s), or other sensors, in communication with the controller 24, to monitor characteristics of the system and/or operate the system 22 in a manner that provides suitable respiratory support. Such characteristics can include gases concentration, flow rate, pressure, temperature, humidity, or other characteristics. The sensors 28, such as pressure, temperature, humidity, and/or flow rate sensors, can be placed in various locations of the system 22 such as for example, inside a main housing containing components of system 22, the patient conduit 12, and/or the patient interface 200. The controller 24 can receive signals from the sensors 28 providing controller inputs used in determining control of one or more components of the respiratory support system 22 in a manner that provides suitable respiratory support. For example, the controller 24 may use signals from one or more of the sensors 28 to determine a suitable target temperature, humidity, flow rate, and/or oxygen concentration of the gases flow, or suitable pressures that may be generated downstream from the flow source 12 within the system. Providing suitable respiratory support can include meeting a patient's inspiratory demand. The suitable respiratory support flow rates, such as a high flow rate, and/or a flow rate meeting or exceeding the patient's inspiratory demand, are explained elsewhere herein.

In some embodiments, the sensors comprise flow rate sensors (such as e.g. ultrasonic, thermal based or other suitable flow rate sensors) configured to sense flow rate of the flow of gases provided to the patient 16, and the sensed flow rate is used by the controller 24 to determine a safe upper pressure limit for operation of the system 22 at the sensed flow rate. The controller 24 can then control operation of one or more components (e.g. actuators) of system 22 (or transmit a control signal to one or more components of the system) such as the flow source 12 to e.g. modify blower speed, so as to achieve a target flow rate stored in the controller, or a memory in operative communication with the controller or any other component of the system. The target flow rate may be a flow rate set point entered by a user of the system through I/O interface 20 or it may be determined or pre-programmed into the controller or associated memory/components. Modifying the flow rate may involve an increase or decrease in blower speed (and/or an increase or decrease in aperture size of a proportional valve), to achieve an increase or decrease in flow rate, respectively.

In some embodiments, when the system begins operation from an 'off' or dormant state, the controller is configured to monitor the flow rate of the flow of respiratory gases in the system and is configured to control the flow modulator to modulate the flow of respiratory gases to achieve the target flow rate. Ideally, the target flow rate changes over time to increase from a current value to a flow rate set point. The controller may also be configured to monitor the flow rate of the flow of respiratory gases to a new target flow rate in other circumstances, such as when a user changes the flow rate set point to which the system operates.

The controller 24 may receive inputs from one or more pressure sensors. The controller can measure or infer the pressure delivered to the patient from the pressure sensor inputs. The pressure sensors are located downstream of the flow source 12. For example, a pressure sensor can be located at or near the patient interface 200. A pressure sensor can also be located directly after the flow source. The pressure delivered to the patient's airways (patient pressure) can be determined by the controller calculating the difference between the ambient pressure and the absolute pressure downstream of the flow source. The patient pressure can be estimated by measuring the pressure in the main device housing downstream of the flow generator and calculating the pressure drop along the delivery conduit 202. The pressure sensor(s) can also be located at other locations in the pneumatic circuit downstream of the flow source 12gases flow. The pressure sensor(s) can include one or more gauge pressure sensors, or alternatively one or more absolute pressure sensors. The gauge pressure sensor(s) can directly measure a difference between the absolute pressure downstream of the flow generator and the ambient pressure. In systems having two absolute pressure sensors, one sensor can be located downstream of the flow source to measure the absolute pressure downstream of the flow source and the other sensor can be located in a different location to measure the ambient pressure. The controller can determine the pressure delivered to the patient by determining the differences between the pressure measurements made by the two absolute pressure sensors.

The controller 24, in various embodiments, is configured to receive one or more signals directly from one or more pressure sensors, or through manual inputs provided to a user interface which is in operative communication with the controller, or derived values, which are indicative of system pressure in the pneumatic circuit downstream of the flow source 12 through which the flow of respiratory gases in the respiratory system 22 are delivered to patient 16. The controller 24 may convert the received signals to pressure values, and the controller may compare these values with pressure threshold values for corresponding flow rates as stored by the controller or a memory device or module in operative communication with the controller. Since the controller is required to know the flow rate of gases in the pneumatic circuit in order to ascertain if the system pressure meets or exceeds the pressure threshold for corresponding flow rates, the controller may also receive one or more signals directly from one or more flow rate sensors, or the controller may obtain flow values through manual inputs provided to a user interface in operative communication with the controller, or it may derive flow values e.g. by knowing the resistance to flow in the pneumatic components of the system 22, and calculating the flow from e.g. blower speed in the flow source 12.

Controller 24 may determine the system pressure continuously, or intermittently and there may be benefits associated with each. For example, continuous determination of system pressure provides for maximum responsiveness in terms of how fast the controller can detect a change in which system pressure meets or exceeds (or falls below) the pressure threshold for a corresponding flow rate, and respond quickly by controlling the flow source 12 e.g. to reduce blower speed (which may in turn reduce the flow of respiratory gases) in response to the pressure meeting or exceeding the pressure threshold values for the corresponding flow rates, and to control the flow source 12 to modulate the flow of respiratory gases in response to the pressure not meeting or exceeding the pressure threshold values for the corresponding flow rates. Alternatively, the controller 24 may sense or receive pressure inputs intermittently which provides for more economical operation, however the system may experience delays in responsiveness since the system pressure may change sometime before the controller receives a pressure input, and so the modulating and/or control of the flow source 12 will not be as responsive.

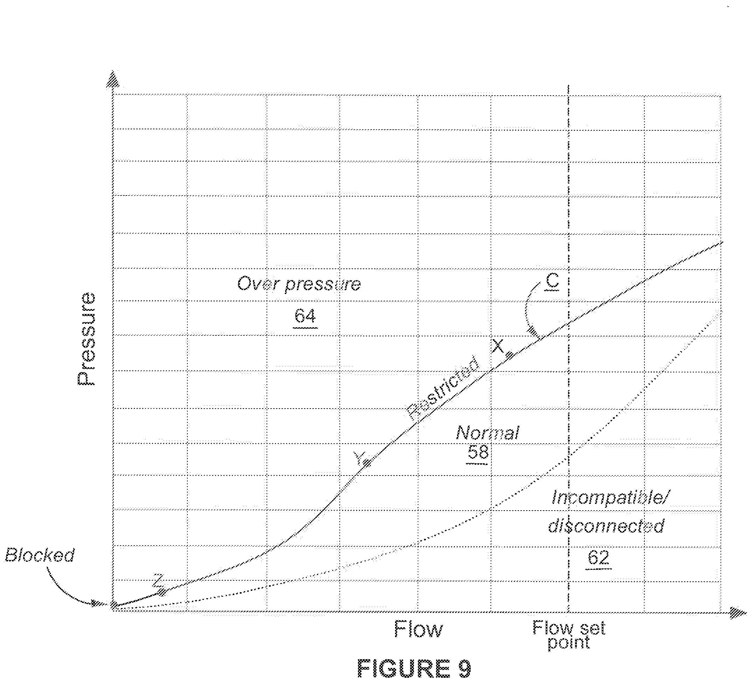

The pressure threshold values for corresponding flow rates may be stored by a memory component of the controller 24 or a memory device or component external to and in operative communication with controller 24. The relationship between the pressure threshold values and the corresponding flow rates may be stored as one or more of a function, a curve, a lookup table, or a mathematical model or algorithm used by the controller to determine the particular pressure threshold value for a corresponding flow rate. In some embodiments, the relationship between the pressure threshold values and the corresponding flow rates as utilised by the controller 24 can be represented graphically and examples are shown in Figures 9 to 14.

Figure 5 shows another embodiment of a respiratory system 22 comprising a controller 24 and a flow source 12 configured to be controlled by the controller 24 to modulate a flow of respiratory gases to a patient 16 via a patient interface 200, in fluid communication with the flow source 12 and configured to deliver the flow of respiratory gases to the patient 16. The respiratory system 22 also comprises one or more sensors 28 configured to determine system pressure downstream of the flow source 12, and/or the flow rate of the flow of respiratory gases in the respiratory support system providing the gases to the patient.

The patient interface 200 receives a flow of respiratory gases from the flow source 12 via a conduit 202. Conduit 202 comprises a first portion 204 configured to operate in at least a first configuration and a second configuration.

The controller 24 in the embodiment shown in Figure 5 may be configured to receive inputs indicative of pressure and/or flow rate of the flow of respiratory gases in the respiratory support system 22 from the sensors 28, compare the sensed pressure with a pressure threshold, and control the flow source 12 to provide a first modulation of the flow of respiratory gases in response to the pressure meeting or exceeding the pressure threshold, and control the flow modulator 26 to provide a second modulation of the flow of respiratory gases in response to the pressure not meeting or not exceeding the pressure threshold.

As mentioned, in some embodiments, the second modulation is different from the first modulation and the first modulation comprises maintaining the system pressure downstream of the flow source at a target pressure, for example at the pressure threshold and/or reducing the system pressure downstream of the flow source to a target pressure that is below the pressure threshold. The second modulation may comprise increasing, or decreasing the flow rate of respiratory gases to a target flow rate in response to the system pressure downstream of the flow source not meeting or not exceeding the pressure threshold.

A corresponding flow rate may be a flow rate of the flow of respiratory gases provided by a flow source, at or along a portion of the pneumatic circuit of respiratory system 22 providing the flow of gases to the patient interface 200, at the patient interface 200 and/or at the patient's airways. As noted in the foregoing, the corresponding flow rate may be a flow rate obtained by one or more flow rate sensors providing input directly to controller 24, it may be entered by a user operating a user interface in operative communication with the controller, or it may be inferred from other factors such as e.g. blower speed of the flow source, if the resistance of the pneumatic components of the system 22 are known.

Figure 6 shows a respiratory system 22 according to another embodiment of the present disclosure. In this embodiment, the system 22 includes the controller 24 and the flow source, in the form of a flow modulator or a blower 27, configured to be controlled by the controller 24 to generate a flow of respiratory gases to a patient 16 via a delivery conduit 202. Typically, the controller 24 and the blower 27 are contained within a common housing 33 although it is contemplated that the controller could be located remotely from or in a separate housing to the blower 27 and other components of the respiratory support system. In some embodiments, the housing 33 also contains a humidifier (as shown in the embodiment of Figure 1) comprising a humidification chamber through which a flow of gases from the blower is passed to humidify the gases provided to the patient by increasing the moisture content and in some cases, temperature. The humidified gases are then delivered by delivery conduit 202 (which may comprise a part of the delivery circuit 38 downstream of the humidifier and the patient breathing circuit 40) to the patient interface 200. The respiratory support system 22 further includes a patient interface 200 configured to receive a flow of gases through delivery conduit 202 for delivery to the patient 16. The patient interface 200 may comprise one or more nasal elements or a face mask assembly configured to provide gases to the patent's nare(s) or mouth respectively or both. The patient interface 200 receives a flow of respiratory gases from the blower 27 via a conduit 202. Conduit 202 comprises a collapsible first portion 204 configured to operate in at least a first configuration and a second configuration as described elsewhere in the specification.

The collapsible portion 204 is configured to operate in a first configuration (e.g. un-collapsed or fully or substantially open condition) and collapsible from the first configuration to a second configuration (e.g. collapsed or fully or substantially closed condition). It will be understood that there may be one or more intermediate conditions between the first and second configurations, where these one or more intermediate conditions may be less open (or more closed) than the fully or substantially open condition (first configuration) but more open (or less closed) than the fully or substantially closed condition (second configuration).. As mentioned, controller 24 alters the operation of the flow source when the collapsible portion 204 is in the second configuration compared to when the collapsible portion 204 is in the first configuration. In some embodiments, this is achieved by modulating (e.g. reducing) the speed of the blower 27 which may in turn modulate (e.g. reduce) the flow of respiratory gases to a reduced flow rate in the system in response to the sensed system pressure meeting or exceeding the pressure threshold values for the corresponding flow rate of the flow of respiratory gases being provided to the patient. In some embodiments, the controller 24 alters the condition (e.g. extent of opening) of one or more proportional valves which may in turn modulate (e.g. reduce) the flow of respiratory gases to a reduced flow rate in the system in response to the sensed system pressure meeting or exceeding the pressure threshold values for the corresponding flow rates of the flow of respiratory gases being provided to the patient. In some embodiments, the controller 24 alters the operation and/or condition of both a blower and a proportional valve.

The respiratory system 22 shown in Figure 6 includes two types of sensors: flow rate sensor(s) 32 configured to sense flow rate of the flow of respiratory gases in the respiratory system 22; and pressure sensor(s) 29 configured to sense pressure in the respiratory system 22 downstream of the blower 27 including downstream of an outlet of the housing 33.

Flow rate sensor(s) 32 and pressure sensor(s) 29 are located downstream of the flow modulator, i.e. blower 27. The flow rate sensor(s) 32 and pressure sensor(s) 29 are configured to sense flow rate and pressure, respectively, downstream of the flow modulator and in the delivery conduit 202. The controller 24, in this embodiment, is configured to receive data indicative of pressure and flow rate of the flow of respiratory gases in the delivery conduit 202, from the pressure sensor(s) 29 and flow rate sensor(s) 32, and to compare the sensed pressure with pressure threshold values for corresponding flow rates as stored by the controller 24 or a memory component with which the controller is operatively coupled.

In some embodiments, one or more pressure sensors may be located downstream of blower 27 near the system outlet to which delivery conduit 202 is coupled, to determine system outlet pressure. The pressure at the outlet can be used to infer patient pressure as discussed below. In some embodiments, a second pressure sensor 29 may be provided for redundancy, to provide a backup pressure input in the event that the primary pressure sensor fails. In some embodiments, the controller 24 may receive an input from an absolute (ambient) pressure sensor 29a to detect operating air pressure which may vary e.g. due to altitude, and can have a bearing on performance of the flow source and its components such as the blower. Gauge sensors, however, may have superior resolution and may be a preferred pressure sensor for many applications.

In some embodiments, in addition to the pressure sensor 29 configured to sense pressure in the delivery conduit, there is a pressure sensor configured to sense pressure delivered to the patient. The controller 24 can then determine the flow rate of the flow of gases to the patient from the sensed difference in pressure between these two sensors..

The patient interface 200 has features as described above. Thus in some embodiments, patient interface 200 is a nasal cannula, such as a non-sealing nasal canula, receiving a flow of respiratory gases from a conduit 202 having a collapsible portion 204 which is capable of transitioning between a first configuration and a second configuration (Figure 3b).

The patient interface 200 will hereinafter be referred to as nasal cannula 200 to provide more particular description of parts involved in the delivery of respiratory gases according to embodiments of the disclosure. In some embodiments, the collapsible portion 204 is collapsed to the second configuration when a face mask assembly 300 is applied to the patient 16 over the nasal cannula 200. The face mask assembly 300 may be referred to in short form as mask 300. The delivery conduit 202 is thus configured to deliver the flow of respiratory gases to the patient 16 through the collapsible portion 204 when in the second configuration at a reduced flow rate compared to when the collapsible portion 204 is in the first configuration. For example, the reduced flow rate may be less than about 15L/min, about 0-15L/min or about 5-15L/min or 0L/min. The flow rate when the collapsible portion 204 is in the first configuration is a high flow rate which may be more than about 20L/min, in the range of about 20-90L/min, or about 40-70L/min.

The delivery conduit 202 may be considered to provide a delivery circuit 38 and a patient breathing circuit 40 disposed between the delivery circuit 38 and the patient interface 200. The patient breathing circuit 40 is connected to the delivery circuit 38 via an outlet connector 34. In the embodiment of Figure 6, the controller 24, blower 27, pressure sensor(s) 29, flow rate sensor(s) 32, and the delivery circuit 38 are housed within a housing 33. The outlet connector 34 may be mounted to or through the housing 33 to provide a physical coupling between the delivery circuit 38 inside the housing and the patient breathing circuit 40 outside the housing. It will be appreciated that the patient interface 200 may be connected to the patient breathing circuit 40 via a connector that is not shown in this Figure. It will also be appreciated that the system 22 may further comprise one or more proportional valves, of the type described above, and this may also be housed within the housing 33.As will be appreciated, intentionally collapsing a portion of the patient interface 200, or the collapsible portion 204 from which the patient interface receives a flow of gas, introduces a restriction to flow in the respiratory system. When this occurs, in an example of the respiratory system 22 in use, the controller 24 detects the restriction to the flow by determining there has been an increase in system pressure, and may control the flow modulator 26 (for example by changing the speed of the blower 27 and/or a size of a flow passage through a proportional valve 25, see Figure 7) to modulate the system pressure. When the collapsible portion 204 returns to the first configuration, the controller 24 detects the change by determining there has been a reduction of system pressure, and may control the blower 27 and/or the proportional valve 25 to increase the flow of respiratory gases which may, in turn, increase the system pressure, while it remains below the pressure threshold corresponding to the flow rate at which the gases flow.

Respiratory support systems may be susceptible to accidental restrictions to flow, caused by e.g. snagging, folding, or crushing of the delivery conduit 202. Another way flow may be restricted in such systems is when specific patient anatomy and/or airway features have higher resistances. These restrictions may create a substantial backpressure in the flow path upstream of the restriction. In some embodiments of the respiratory system 22, when these restrictions occur, the respiratory system may determine by operation of controller 24, that pressure in the delivery conduit 202 exceeds the pressure threshold value for a corresponding flow rate and the controller 24 can control the blower 27 to reduce the flow of respiratory gases - thus reducing pressure in the system 22. When the accidental restriction is removed, the controller 24 may further control the blower 27 to increase the flow of respiratory gases - thus increasing pressure in the system 22. Alternatively or additionally the controller 24 may control proportional valve 25 to open further, thereby increasing the flow of respiratory gas which may increase pressure in system 22.

Figures 7 and 8 are examples of schematic diagrams of components of the respiratory system 22 that provide the flow of respiratory gases to the patient according to embodiments of the present disclosure. More specifically, Figures 7 and 8 show the components in relation to the respiratory system 22 that deliver respiratory gases from an Oxygen (O₂) source 42 (e.g. a wall source or an O₂ tank) and an ambient air source 44 to the nasal cannula 200 and to the patient.

The respiratory system 22, shown in Figures 7 and 8, includes a pressure sensor 46 (e.g. O₂ pressure sensor) configured to sense pressure in an O₂ delivery circuit 47 of the respiratory system 22. The pressure sensor may be used to determine that the O₂ delivery circuit 47 has been connected to an O₂ source 42. Control of the flow of O₂ may be provided by a proportional valve 25 that is controllable by the controller 24 (not shown). The O₂ delivery circuit 47 further includes a flow rate sensor 48 (e.g. O₂ flow rate sensor) configured to sense flow rate of the flow of O₂ from the O₂ supply after it has been modulated by the proportional valve 25.

The respiratory system 22, shown in Figures 7 and 8, further includes flow rate sensor 50 (e.g. air flow sensor) configured to sense air flow rate from the ambient air source 44 in an air delivery circuit 49 of the respiratory system 22. In some embodiments patient breathing circuit 40 is connected to the O₂ delivery circuit 47 and the air delivery circuit 49 and the patient breathing circuit and/or the patient interface further comprises a patient pressure sensor and a patient flow rate sensor. Control of the flow of air is provided by a blower 27 of the type described above that is controllable by the controller 24. In Figure 7, the blower 27 is provided in the air delivery circuit 49 whereas in Figure 8, the blower 27 is provided in the patient breathing circuit 40. In both embodiments, the respiratory system 22 provides a closed loop control of pressure and/or flow by the controller 24 controlling operation of the blower 27, e.g. by changing angular velocity/motor speed of the blower 27 and/or current or voltage supplied to the blower 27, and/or by controlling degree of openness of the proportional valve 25 e.g. by changing the current supply to the valve, to limit or alter O₂ entering the patient breathing circuit 40.

In some embodiments, blower 27 provides for modulation of the flow of respiratory gases and proportional valve 25 provides for control of oxygen concentration in the respiratory gases. Both are under control of controller 24. Thus, when a user operates I/O interface 20 to increase flow from a given flow rate set point, control to blower 27 causes its speed to increase which may cause dilution of O₂ concentration. Controller 24 compensates for this by operating proportional valve 25 to open further (e.g. by increasing current to the proportional valve) to allow more O₂ to flow to the patient in order to meet the O₂ concentration set point required by the user. If the user changes the O₂ concentration set point to a higher concentration, the controller 24 will control proportional valve 25 to allow more oxygen to flow into the respiratory gas delivered to the patient.

The patient breathing circuit 40, in the embodiments shown in Figures 7 and 8, is in fluid communication with the O₂ delivery circuit 47 and the air delivery circuit 49. As mentioned, in Figure 8, the patient breathing circuit 40 includes the blower 27 that is controlled by the controller 24 to drive a gases flow, typically comprising air and O₂ to the nasal cannula 200. In both embodiments, the patient delivery circuit 40 may include a patient pressure sensor 54 and/or a patient flow rate sensor 52 configured to monitor pressure and/or flow rate of the combined O₂ and air respiratory gases delivered to the patent via the nasal cannula 200.

Relevantly, in the embodiments of Figures 7 and 8, there may be an open flow path in the air delivery circuit 49 (for example between the inlet of blower 27 which receives ambient air from source 44, and the locations in the flow path downstream of the blower 27 in Figure 7) which permits bi-directional flow. An open flow path includes a flow path devoid of one-way valves or components that only permit a single directional flow of gases. This may be beneficial in scenarios where there is a blockage e.g. at an outlet of the system that couples with the patient breathing circuit 40, so that excess gas build up in the system can safely "leak" out of the air delivery circuit 49 through the blower 27 avoiding high system pressures that could damage some components. Many prior art respiratory support systems rely on one way valves to deliver flow to the patient and these preclude backflow in the circumstances described, potentially giving rise to component damage and/or system failure.

In the embodiment of Figure 7, O₂ is blended with ambient air downstream of the blower 27. This may be beneficial for embodiments that provide humidification of gases provided to the patient, since gases blended downstream of the blower 27 may have a lower temperature than gases blended upstream of the blower (as in Figure 8) as operation of the blower would heat up gases passing through it. This may give rise to improvements in humidification accuracy when control of the humidifier is based on temperature of gases entering the humidification chamber. In some embodiments, one or more pressure sensors may be provided to monitor ambient air pressure to enable adaptation to different operating conditions, such as changes in altitude.

Figures 9 to 14 are graphs illustrating curves that represent a relationship between flow and pressure, which are used in embodiments of the present disclosure in the control of components of a respiratory system to deliver a flow of respiratory gases to a patient. As mentioned, the controller 24 is configured to control the flow modulator 26 to increase, maintain or reduce the flow of respiratory gases . For example, the controller 24 is configured to control the blower 27 to increase, maintain or reduce an angular velocity (i.e. speed) of the blower 27 by providing an electronic control signal, or by directly controlling the voltage or current supply to the blower, which in turn increases, maintains or reduces the flow of respiratory gases. The controller 24 determines the appropriate control for the flow modulator 26 in response to the pressure not meeting or not exceeding, or meeting or exceeding, one or more pressure threshold values at corresponding flow rates, such as those represented in the curves of Figures 9 to 14 which may hereinafter be referred to as pressure limit curves C. In some embodiments, the controller 24 determines the appropriate control for the flow modulator 26 in response to the system pressure being below or meeting or exceeding, a pressure threshold value at a corresponding flow rate. One or more pressure threshold values may be a predetermined pressure threshold value. As mentioned, the pressure threshold values and corresponding flow rates may be stored in a memory component of or operatively coupled with controller 24.

In some embodiments, the controller 24 is configured to control the flow modulator to modulate the flow of respiratory gases to the target pressure when the flow rate of the flow of respiratory gases is less than the target flow rate. This avoids a condition where the controller tries to increase flow to approach the target flow rate which may cause the system pressure to exceed the pressure threshold value for the current flow rate. Alternatively/additionally, the controller may be configured to control the flow modulator to modulate the flow of respiratory gases to the target flow rate when the flow rate of the flow of respiratory gases is above the target flow rate, i.e. to modulate flows down to the target flow rate when it has been exceeded, to avoid risk of harm to the patient. In some embodiments, the controller is configured to control the flow modulator to the target pressure when the pressure of the flow of respiratory gases exceeds the pressure threshold value corresponding to the target flow rate or a pressure threshold value corresponding to flow rates less than the target flow rate.

The pressure limit curves C have been determined by taking into account known resistances to flow of system components and known flow ranges to be delivered to a patient. It will be appreciated that the pressure limit curves C may be stored in a memory that is accessible by the controller 24 as discussed previously and which may include a computer-readable medium (e.g., a disk, hard drive, USB, optical drive or other data storage device) containing program instructions for causing the controller 24 to perform the necessary control as described herein.

In some embodiments, a pressure limit curve C provides a safety threshold corresponding to a safe patient pressure for a corresponding flow rate of gases to the patient when there is restricted flow to the nasal cannula 200, such as when collapsible portion 204 is in the collapsed configuration and a mask is applied. This pressure threshold is applied to reduce risk of over-pressurising the patient, which may lead to barotrauma of the patient's airways. Thus, in some embodiments, the controller 24 automatically reduces flow through the nasal cannula 200 to the patient when there is a blockage in the delivery conduit 202 such as an accidental blockage, or when the mask 300 is used to ventilate the patient and is applied over the nasal cannula with a pressure sufficient to collapse the collapsible portion 204 e.g. to the second configuration. When this occurs, the controller 24 may implement pressure-control of the system 22 by controlling operation of the blower 27 and/or other components such as a proportional valve 25 to achieve a target pressure guided by pressure limit curve C. Further, controller 24 may automatically increase the flow of respiratory gases when the mask is removed enabling the collapsible portion 204 to return to the first configuration.

In some embodiments, the controller 24 may control only the blower 27 (e.g. angular velocity), the blower 27 and one or more proportional valves 25 or only one or more proportional valves 25, to control a flow rate and/or pressure of the flow of respiratory gases. In embodiments where a proportional valve and blower are in parallel flow paths (for example Figure 7), a non-return valve (not shown) may be provided before or after the blower to the air delivery circuit 49 to prevent reverse flow out of the air delivery circuit 49 via ambient air source 44, such that flow and/or pressure of the flow of respiratory gases is controlled by the proportional valve. In some embodiments, a pressure relief valve (not shown) may be provided to the system, where the pressure relief valve (for example one that vents to atmosphere) is operable by the controller 24 to control the flow and/or pressure of the flow of respiratory gases.

In some embodiments, the controller 24 may be programmed to apply a further safety threshold being a pressure limit which, when reached at any flow rate, will trigger the controller 24 to substantially reduce or stop operation of the flow modulator 26, for example by turning off the blower 27 and/or closing off of the proportional valve 25. This safety threshold may be a value above about 20 cmH₂O such as for example about 30 cmH₂O, about 40 cmH₂O, about 50 cmH₂O or about 60 cmH₂O or about 70 cmH₂O. In some embodiments, a safety threshold of about 60 cmH₂O may be preferred, or a safety threshold calculated as a safe margin above the predetermined pressure threshold value for the corresponding flow rate may be used, or calculated as a safe margin above a normal expected region or range of system pressures.

The resistance to flow of system components may be measured, calculated and/or estimated, and may include the resistance of all components of the respiratory support system 22, optionally all components downstream of an outlet of the flow modulator 26. This requires carefully designed consumables of the system 22 to have tight tolerances for resistance to flow, or use of conservative assumptions to account for less strict manufacturing tolerances. Knowledge of the resistance of the system 22 enables the estimate of the pressure difference across the respiratory system 22 to allow inference of patient pressure from system pressure. The respiratory system 22 may also determine a real time characterization of resistance in the delivery conduit 30 from the pressure sensors 29 and flow rate sensors 32, compare this to the known resistance to reduce expected variation in the patient pressure. Knowledge of the current patient pressure ensures pressure can be controlled according to the pressure limit curve C and, as a result, the respiratory system 22 can better control pressure/flow to the nasal cannula 31. In practice, controller 24 operates the blower 27 and other components of system 22 to avoid or minimise operation of the system in the "over pressure" area corresponding to region 64 as discussed below.

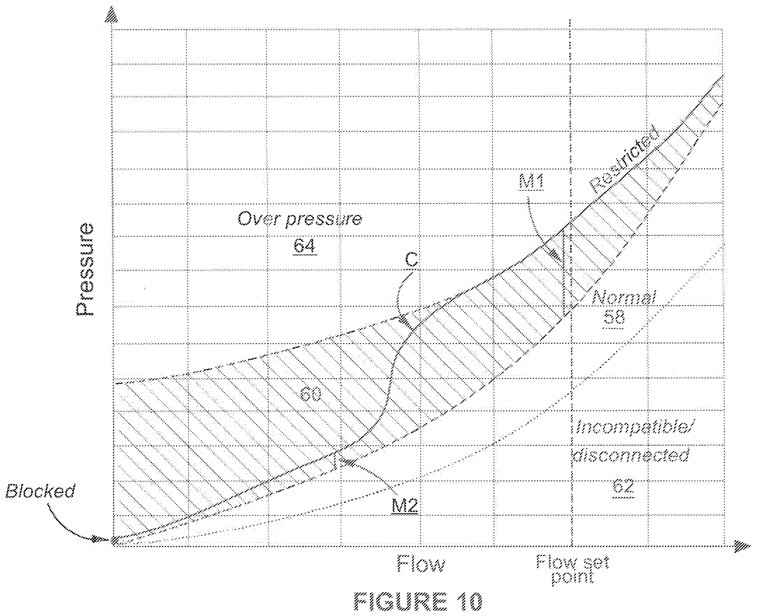

During operation of the respiratory support system 22, the "normal" operating pressure values for corresponding flow rates is shown as being beneath the pressure limit curve C. More specifically, Figures 9 and 10 show an area 58 corresponding to Normal operating system pressures at corresponding flow rates. Advantageously, the Normal operating zone provides a range of safe system pressures for the corresponding flow rates and therefore provides a "safe" zone which in practice provides scope for a clinician to safely manipulate components of the system, such as the delivery conduit 202, collapsible portion 204, manifold 206, cannula 200 and other components required for delivery of gases to the patient, without risk of delivering excess pressure to the patient's airways and risking barotrauma or other harm.

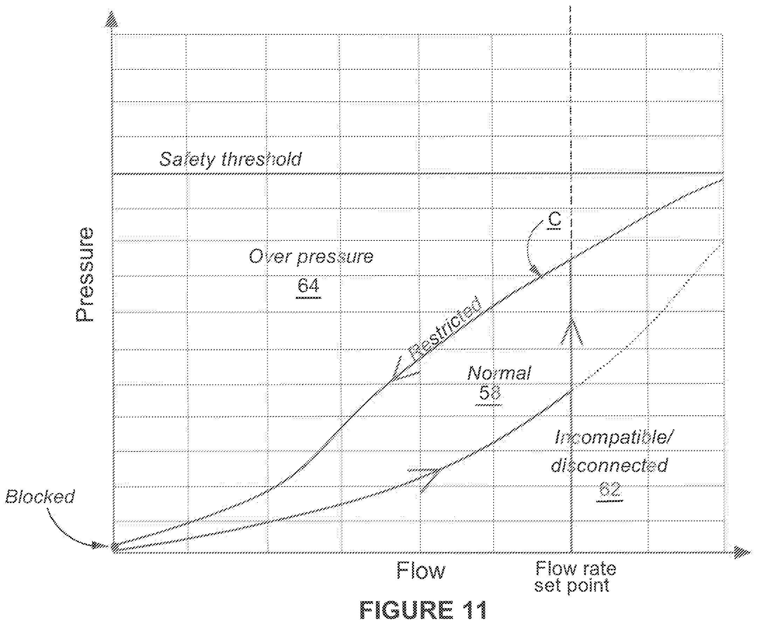

Figure 10 shows a restricted operating area 60 in which a pressure limit curve C may be defined for use in controlling operation of the system for delivery of respiratory gases to the patient. In some embodiments, the controller calculates the shape of curve C using a function stored in a memory component of or operatively coupled with the controller. Thus, upon receipt of a "set point flow rate" entered to the I/O interface 20 by a user, the controller defines the upper flow limit of the required curve C and, knowing the lower pressure limit (which may be defined by the manufacturer or the user as an operational constraint of the system), uses the function to fit the curve within the restricted operating area 60. When the controller 24 determines the system pressure to be within the restricted operating area 60 and under the curve C it may not immediately alter control of the system components to reduce flow rather, it may permit continued delivery of flow at the current control. The restricted operating area 60 may be desirable to avoid the controller 24 immediately reducing flow rate every time there is a bend in the tube which may be inadvertent, or may occur as clinicians are interacting with the patient and/or arranging various components of the respiratory support system including patient interfaces on the patient causing temporary or transient blockages or restrictions to flow. This area is absent from the embodiment shown in Figures 9 and 11 as the pressure limit curve C has been defined immediately next to the normal operating region and provides the pressure thresholds that may be used by the controller. The control parameters conveyed by embodiment of Figures 9 and 11 may be utilised in scenarios where it may be desirable to limit the extent of inadvertent (or deliberate) temporary blockages in the delivery conduit 202 before flow rate is reduced.

The area below the pressure limit curve C and below the normal operating area is the incompatible area 62. Operation in this area might occur if the delivery conduit leaks or becomes disconnected from the flow generating components of the system, e.g. if it becomes decoupled from the housing 33. Flow rates and pressures in this incompatible area 62 may also occur if incompatible or unintended system components are used. This area may also include pressures for corresponding flow rates which may be undesired, such pressures that may not achieve adequate or intended respiratory support. In some embodiments, detection by controller 24 of pressures in the incompatible/disconnected zone 62 may result in controller 24 sounding an alarm or alert, which may be visible and/or audible, which communicates to system users that the system has detected an incompatible condition for example one that may signify a leakage or disconnection in the system 22.

The area above the pressure limit curve C, and above the restricted operating area 60 shown in Figure 10, is referred to as the over pressure area 64. In embodiments that apply a safety threshold (Figure 11), this limit may be located within the over pressure area 64 .When the controller 24 determines that the system pressure exceeds the threshold defined by pressure limit curve C, it may alter control of the blower 27 to reduce the blower speed in order to lower system pressure to a target pressure, for example a pressure at or below a pressure value defined by curve C at the corresponding flow rate. Alternatively/additionally, the controller 24 may control operation of a valve such as a proportional valve 25 to lower pressure in the system. In some embodiments, the controller 24 may be configured to track time spent in the restricted operating area 60 and/or above the pressure limit curve C and, if the pressure in the respiratory system 22 remains in this area after a certain period of time, the controller 24 may trigger an audible and/or visible alarm to alert users to the possibility of a blockage in the gas delivery conduit or other components of the system.

Figure 10 also shows that the sigmoidal pressure limit curve C allows for a temporary increase in pressure to occur without that increase causing the controller to reduce pressure and/or flow. That is, in use, in the first configuration of the collapsible portion 204, a nominal (baseline) system pressure in the normal operating pressure area 58 is below the pressure limit curve C by a first pressure margin M1 or greater. In the second configuration of the collapsible portion 204, a nominal (baseline) system pressure in the normal operating pressure area 58 is below the pressure limit curve C by a second pressure margin M2 or greater. The nominal (baseline) pressure may be an upper pressure limit of the normal operating pressure area 58. It can be seen in Figure 10 that the first pressure margin M1 is greater than the second pressure margin M2. That is, the system 22 provides an allowable deviation of pressure change in the system 22. When the system 22 is in the normal operating state 58, there is a larger margin of allowable deviation from normal system pressure at higher flows than when the flow is lower. The tight pressure margins at lower flows which may correspond to the collapsed state and the greater pressure margins at higher flows which may correspond to the uncollapsed state provide that in the absence of a significant pressure and/or flow rate change, the control of system 22 remains in those regions and in the "collapsed" or "uncollapsed" states respectively. Accordingly, the controller 24 requires pressure in the system to vary significantly before initiating a change in control (e.g. from a pressure control to flow control or vice versa) as may be required when the collapsible portion 204 transitions from a second (collapsed) configuration to a first (uncollapsed) configuration or vice versa, ensuring that control is not altered until the blockage is substantially unblocked (triggering a shift to flow control) or until the blockage is more than transient (triggering a shift to pressure control).

Referring to the graph of Figure 11, during operation of the respiratory support system 22 in the normal operating pressure region 58, the controller 24 is configured to control the flow modulator 26 to provide a flow of respiratory gases at a target flow rate, for example a set point flow rate prescribed by the user such as the clinician using I/O interface 20. As long as the system pressure remains beneath the pressure value defined by pressure limit curve C at that target flow rate, in the normal operating area 58, the controller 24 controls the flow modulator to provide a flow of gases at that target flow rate. In such a situation, the controller may be considered to be in a first control mode which is a flow rate control mode. According to the control illustrated in Figure 11, when the controller 24 determines that the system pressure meets or exceeds the pressure value defined by the pressure limit curve C at that target flow rate (for example when an obstruction starts to occur), the controller 24 is configured to control the flow modulator 26 to modulate the flow of respiratory gases to a target pressure which is at or below the pressure value defined by the pressure limit curve C. In such a situation, the controller may be considered to be in a second control mode which is a pressure control mode. In the embodiment of Figure 11, the target pressure comprises a pressure value along pressure limit curve C. As the obstruction increases (for example collapsible portion 204 becomes more collapsed), flow rate of the flow of respiratory gases in the system reduces and the corresponding target pressure at the reduced flow rate also reduces. Accordingly, the controller 24 controls the flow modulator 26 to modulate the flow of respiratory gases downwards along pressure limit curve C when the amount of obstruction increases.

While the controller 24 may be configured to identify the system pressure as "meeting" the pressure limit curve C when the sensed pressure input value equates to the value of the curve for the corresponding flow rate, it is to be understood that the controller may also be programmed to activate a change in control when the sensed system pressure "approximates" or is very close to the value in the pressure limit curve C such as e.g. within up to about 10% of the curve value, or within up to about 5% of the curve value, or within up to about 3% of the curve value, or within up to about 2% of the curve value or within up to about 1% of the curve value. In some embodiments, the flow rate of the flow of respiratory gases may be reduced progressively and could eventually reach a very low flow rate approaching (or in some cases reaching) 0 L/min. This reduces pressure in the respiratory system 22 and may avoid pressure in the system 22 from meeting or exceeding the pressure limit curve C.

In some embodiments, the controller 24 controls the flow modulator 26 to control the flow of respiratory gases to maintain the system pressure at or along the pressure limit curve C. If the system pressure falls below a pressure limit along the pressure limit curve C at a corresponding flow rate, the controller 24 may control the flow modulator 26 to increase the flow of respiratory gases to achieve a target pressure. This target pressure may be a pressure value along pressure limit curve C. This target pressure may be a higher target pressure along pressure limit curve C. In other words, as an obstruction reduces or as the bower increases speed for a given system pressure, the flow rate of respiratory gases increases and the corresponding target pressure along pressure limit curve C increases, such that the controller controls the flow modulator 26 to modulate the flow rate of respiratory gases upwards along the pressure limit curve C as the obstruction reduces, towards the flow rate set point. The control to a target pressure may be switched to a flow rate control when the target flow rate is met, optionally where the target flow rate is met and the system pressure is less than the pressure value along pressure limit curve C at that target flow rate. The increase and/or reduction in the flow of respiratory gases may be a continual increase and/or reduction or it may be a stepped increase and/or reduction.

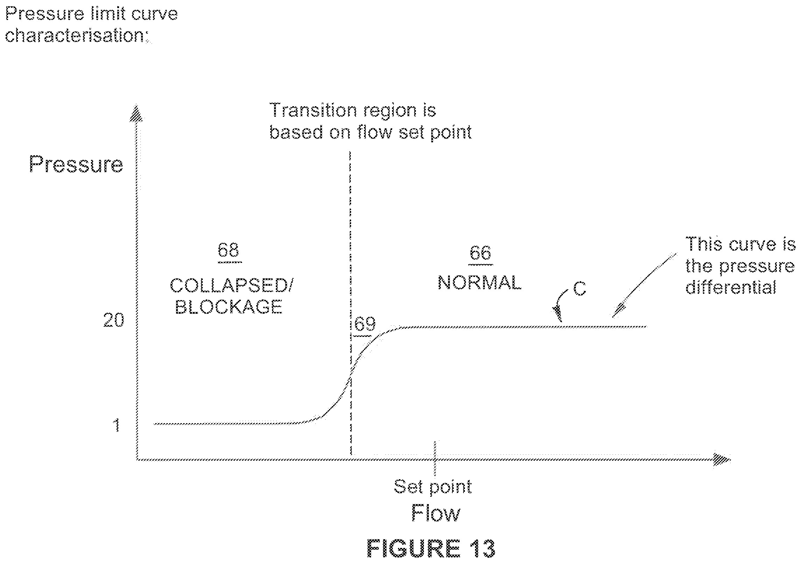

The pressure limit curve C has been devised to provide guidance to the controller 24 to avoid or reduce the likelihood of restricted flow in the system 22 causing an over pressure event or condition which may deliver unsafe patient pressures or system pressures that could damage system components. As mentioned, the restriction may be caused intentionally by collapsing of the collapsible portion 204 supplying or forming part of the nasal cannula 200 with application of a mask 300 or the restriction may be caused accidentally by, for example, snagging, kinking or bending a portion of the delivery conduit. The shape of the pressure limit curve C in Figure 10 and 13 has a sigmoidal or "S" shape. In embodiments utilising a sigmoidal pressure limit curve, the controller 24 may operate the flow modulator 26 to modulate the flow rate of gases by a variable rate of increase or decrease. A sigmoidal pressure limit curve C allows for the controller 24 to control the flow modulator to provide or maintain a low system pressure at low flows. This may be beneficial in enabling the collapsible portion 204 to remain collapsed while also minimizing the chance of there being a pressure difference or significant pressure difference across the collapsible portion when in the second configuration which may otherwise drive residual flow through the collapsed portion 204 to the patient, or make it difficult to apply a mask over a cannula for delivery of a required respiratory support. In some embodiments, the pressure limit curve C may be defined such that a known amount or range of amounts of residual flow across a collapsible portion 204 in the second configuration is achieved. For example, by providing a certain amount of pressure differential across the collapsible portion 204 in the second configuration to achieve a certain amount of residual flow. The smoothness of the pressure threshold curve C provides for smooth tactile feedback to the user who will be able to feel, through the mask 300 if there is a force being applied by the system pressure, through the collapsible portion 204 and the mask 300.

It is to be understood that the pressure threshold curve C may have a variety of shapes however in all circumstances, it is shaped to direct control to achieve delivery of gases at or below a pressure limit for the corresponding flow rates, and is ideally relatively smooth to achieve smooth control (although stepped control arising from a stepped curve C may also be adopted in some embodiments however these may give rise to control instability between steps). The normal operating region 58 beneath the curve C may be determined by a resistance to flow of components within system 22, allowing for different manufacturing tolerances as discussed above. Parameters of curve C may be varied according to operational preferences. In some embodiments, a higher pressure limit at zero flow (y-intercept), may give rise to greater residual flows across the collapsible portion 204 when in the second configuration. In other embodiments, the target pressure at zero flow may be close to or at 0 cmH₂0. Alternatively/additionally, a higher pressure limit at a high flow rate (i.e. a greater difference between the pressure limit curve C and a normal operating system pressure within region 58) may accommodate more variation in system condition (e.g. state of cannula collapse or blockage) before the controller 24 modulates control of the flow modulator to reduce pressure. This provides a greater tolerance for the system pressure to increase before the pressure limit value in the pressure limit curve C at a target flow rate is met or exceeded. In some embodiments, curve C could be or include a linear portion between the control end points defined by the minimum and maximum operating pressures. However controlling to a linear curve C may give rise to an undesirable amount of residual flows across the collapsible portion 204 when in the second condition at low flows. Alternatively/additionally, curve C could be or include a quadratic portion however this may in some circumstances more easily give rise to accidental triggering of pressure threshold detection in cases where there is an inadvertent or temporary bend or kink or other condition causing a high system pressure. Thus, it has been determined that a sigmoidal or other s-shaped curve, in conjunction with a "restricted" operating area 60 may be preferred for guiding control in some embodiments.

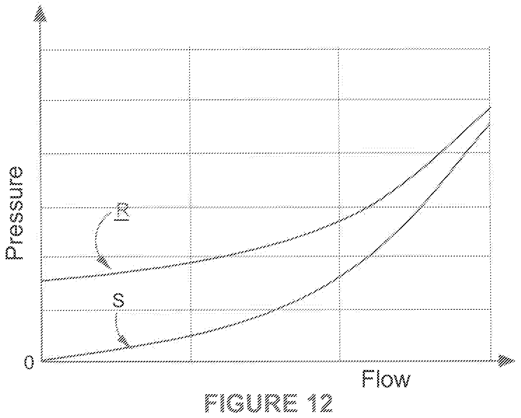

Figure 12 is a graph provided to illustrate that at zero flow the pressure curve can have a nominal pressure limit (curve R) or a zero pressure limit (curve S). Zero pressure limit at zero flow theoretically gives zero residual flow when there is a blocked or collapsed condition in the system. The higher the nominal zero flow pressure limit (curve R), the more residual flow may occur when collapsed. In some embodiments, it may be desirable to operate the system 22 with a small amount of residual pressure at low or zero flow (e.g. when the collapsible portion 204 is in the second condition). In such embodiments, the controller 24 controls and maintains operation of the flow modulator 26 such that a small amount of system pressure is maintained at zero (or low) flow, for example, the blower 27 is maintained at very low angular velocity. This has the benefit of helping to restore flow within the system and to the patient when the collapsible portion 204 changes towards the first configuration. In particular, when the collapsible portion 204 changes from the second configuration to the first configuration (i.e. obstruction is being reduced), the flow rate of the flow of respiratory gases will increase and the system pressure will drop. The controller 24 will control the flow modulator 26 to modulate the flow of respiratory gases to a new target pressure for that given increased flow rate. In trying to achieve that new target pressure, the flow rate of the flow of respiratory gases increases again which provides a new target pressure.

Accordingly, the controller 24 controls the flow modulator 26 to modulate the flow of respiratory gases to a target pressure along pressure limit curve C until the flow rate set point is met, upon which the controller 24 switches to a flow rate control mode. However a small amount of system pressure at zero (or low flow) may cause a pressure differential across the collapsible portion 204 which may result in a residual flow. In some applications, a residual flow is desirable. In other applications, a residual flow is undesirable and such residual flow may be stopped or substantially reduced by configuring the pressure limit curve to intersect the y-axis of the pressure-flow graph at the origin (0,0), which would correspond to a complete shut off of the flow modulator 26. In such an embodiment, the absence of any flow in the system may make it impossible for the controller 24 to determine if there has been a change in pressure and/or flow in the system sufficient to operate the controller 24 in a pressure control (pressure-guided control) and/or flow control (flow-guided control) mode. Accordingly, the controller 24 may be configured to control the flow modulator 26 to deliver short bursts or pulses of flow corresponding to a "test pulse" to determine if there has been a change in the system pressure indicating that the obstruction or blockage (e.g. arising due to the collapsible portion 204 being in the second configuration) has been cleared. If the test pulses give rise only to an increase in system pressure (with negligible increase in flow rate), the controller determines that the obstruction or blockage remains and pressure-guided control (or deactivation of the flow modulator except for test pulse) should remain. The duration between test pulses may be pre-programmed and/or altered according to operational requirements, recognising that more regular (or longer) pulses may increase residual flows if the blockage has not been cleared. Conversely, if the duration between pulses is long, the residual flow may be reduced significantly however the controller may not be sufficiently responsive since a reduction in system pressure cannot be detected until a test pulse occurs. If the controller detects an increase in flow in response to a test pulse, it may determine that the blockage has been cleared and modulates control of the flow modulator to increase speed thereby increasing flow and/or pressure in the system.

In an example, the controller 24 continuously receives or periodically samples data indicative of pressure and/or flow rate of the flow of respiratory gases in the system 22 and compares the received data with the values obtained from pressure limit curve C. Ideally, the controller 24 seeks to achieve the target flow rate corresponding to the flow rate set point, subject to pressures in the system not exceeding a target pressure corresponding to values along curve C. The controller 24 may control the flow modulator 26 according to the received pressure values. For example if the received pressure values indicate that the system pressure meets or exceeds the value in curve C for the corresponding flow rate, the controller 24 controls the system to achieve a system pressure which is at or below the value in curve C for the corresponding flow rate (i.e. pressure guided control). Alternatively, when the received pressure values indicate that the system pressure does not meet or exceed the value in curve C, the controller controls the system to achieve a target flow rate (i.e. flow rate guided control). By way of example, the controller 24 may modulate operation of the system 22 to reduce the flow of respiratory gases in response to the received pressure exceeding the value in pressure limit curve C for the corresponding flow rate by controlling the flow modulator 26 to reduce or stop the flow of respiratory gases being provided to the patient. Alternatively or additionally, the controller may operate a proportional valve 25 to reduce pressure in the system. Conversely, the controller may modulate operation of the system 22 to increase the flow of respiratory gases in response to the received pressure not exceeding the value in the pressure limit curve C for the corresponding flow rates by controlling the flow modulator 26 (and optionally, a proportional valve 25) to increase the flow of respiratory gases in response to the pressure not exceeding the pressure limit curve C for corresponding flow rates.

If the controller 24 determines that the received pressure does not exceed the pressure limit curve C and the controller 24 determines a difference between the target flow rate and a measured flow rate, the controller 24 can then output a command such as by a digital communication interface or by modulating a supply voltage or current, to increase or decrease the motor speed of the blower 27 and/or open or close the proportional valve 25. The control may be binary (e.g. switching the blower on/off) or continuous e.g. by controlling supply voltage or current to the blower and/or the proportional valve to modify the angular velocity of the blower, and the degree of openness of the valve, based on the difference. If the sensed pressure value meets or exceeds the pressure limit curve C, the controller 24 may decrease the supply voltage or current to the blower by a set amount, a set rate of decrease, a variable rate of decrease, or using a pressure-based PID control or the like as described previously. The angular velocity of the blower may be decreased at each iteration of the feedback control until the sensed pressure value is at or below the pressure limit curve C or until a flow threshold is reached. This may indicate a blocked state. The angular velocity of the blower may be reduced at a constant rate or variable rate.

Figure 13 shows an example of a sigmoidal pressure limit curve C in an offset pressure characterisation for improved clarity. It shows the normalised system pressure and how the pressure threshold value at low flows (e.g. 1 cmH20) differs from the pressure threshold value at high flows (e.g. 20 cmH₂0). The sigmoidal shaped pressure limit curve C has a first pressure region 66 corresponding to the normal state of the nasal cannula 31, a second pressure region 68 corresponding to the collapsed state of the nasal cannula 31, or some other restriction of flow in the system 22 or delivery conduit 30. The sigmoidal shaped pressure limit curve C has a transition region designated by broken line 69 which is disposed between first pressure region 66 and the second pressure region 68. The first pressure region 66 is substantially parallel to the second pressure region 68 of the pressure limit curve C. The first pressure region 66 and the second pressure region 68 correspond to pressure thresholds for corresponding flow rates in the respective regions. .The transition region may be determined according to required operational parameters, and is typically dictated by a trade-off between whether it is preferred to enjoy the benefit of low pressure threshold values at a wider range of low flows, or the benefit of higher pressure threshold values at a wider range of high flows. Being joined by a smooth "S" shaped transition provides for smoother control, avoiding oscillations and/or instability that may arise in the system if the pressure transition was an instantaneous step. While two pressure regions have been described in relation to the pressure limit curve C, it is to be understand that additional pressure regions may be provided.

The transition region 69 of the pressure limit curve represents a maximum rate of increase/decrease in the control of the flow of respiratory gases and is centred about a transition flow rate that is less than the flow rate set point (i.e. target flow rate) during normal operation. That is, the controller 24 is configured to control the flow modulator 26 to reduce the flow of respiratory gases at the maximum rate of decrease when the sensed pressure and flow rate is in the transition region 69 of the pressure limit curve and the sensed pressure exceeds the pressure limit curve C for the corresponding flow rate. In an example, the system 22, when in this transition region 69, prevents patient airway pressure from exceeding safe limits and reduces flow (ideally down to near zero) when, for example, the nasal cannula 200 has been collapsed for bag mask ventilation.

As mentioned, the difference between the flow rate at the transition region and target flow rates allows for a temporary increase in system pressure enabling the controller 24 to reduce the flow of respiratory gases at an initial minimum rate of decrease until the transition flow rate is approached in case the cause of the pressure increase is a temporary restriction and the temporary pressure increase is quickly removed.

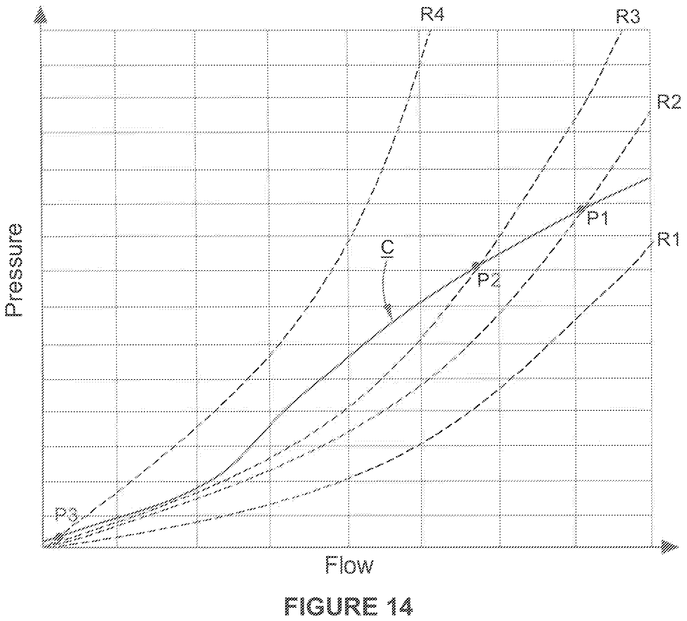

Figure 14 is a graph provided to illustrate the changing relationship between pressure and flow rate as an increasing resistance to flow is experienced by the system e.g. as a clinician applies increasing pressure to a mask applied over a cannula (or when there is an increasing blockage of another type in the system). As the clinician presses down on the mask, the cannula begins to collapse and the resistance to flow in the system increases, detected as an increase in system pressure. The increasing resistance to flow is represented in Figure 14 as the broken lines having increasing slope from R1 to R4, with R1 representing the lowest resistance and R4 representing the highest resistance on the graph. These demonstrate that for a given flow rate, increasing the resistance to flow by collapsing the cannula causes system pressure to increase and with a sufficiently high resistance to flow (e.g. a fully collapsed cannula), the system pressure will intersect with the pressure limit curve C. Higher resistances cross the pressure limit curve C lower down the curve as shown by intersection points P1, P2 and P3. P3 corresponds to a fully or almost fully collapsed cannula (or complete or near-complete blockage of another kind) having high resistance to flow (R4 curve) readily intersecting the pressure limit curve C at low pressure and flow.

In some embodiments, when the controller 24 determines that system 22 has encountered a blockage or the collapsible portion is in the second condition, a collapsed state 68 (Figure 13) has been reached and the controller 24 controls flow modulator 26 to reduce the flow. The controller 24 may further be configured to provide an output such as a visible or audible alert, by I/O device 20 or any other system component, to indicate that the collapsible portion is in a collapsed state and/or delivery of anaesthetic agents may commence. This output may, in some embodiments, include a control signal provided to an anaesthetic machine. This may be beneficial in circumstances when anaesthetic agents are being delivered via inhalation through a mask 300 over nasal cannula 200 because, if flows being provided to the patient are not substantially reduced, these flows can dilute the anaesthetic agents.

As mentioned, Figure 10 shows a restricted area 60 where the sigmoidal pressure limit curve C may be defined, and where a temporary increase in pressure may be permitted to occur before controller 24 alters control of the flow modulator 26. This temporary increase in pressure may occur, for example, due to an inadvertent bending of a tube of the respiratory system 22. In some embodiments, the pressure limit curve C may be set to be as close as possible to the expected normal pressure to minimise flow at lower pressures and allow for movement of mask 300 over the nasal cannula 200 without rapidly ramping up flow to the flow rate set point.

It will be appreciated that the pressure limit curve C is not limited to a sigmoidal shape. The sigmoidal shape, however, allows for a quick transition in pressure between two pressure regions without an instantaneous jump between the two regions. An instantaneous jump may not be ideal as this discontinuity could lead to instability in the control of the respiratory system 22. For example, the output (e.g. blower speed control parameter) from controller 24 would change by large amounts that correspond with the instantaneous rate of change of pressures and flows in the transition region, and this instantaneous rate of change in the controller output will lead to instability..

Referring again to Figure 13, while the respiratory system 22 is operable in normal operating region 66, when operating closer to the transition region 69 of the sigmodal pressure limit curve C, the respiratory system 22 may more quickly transition between pressure states (i.e. higher pressure offset vs lower pressure offset relative to normal operating pressure). As alluded to previously, curves of different overall shapes are envisaged, such as a "Z" shaped curve, which still allows for a quick transition in pressure. The "Z" shaped curve allows for the pressure limit curve to be 'flat' (steady at the pressure limit) in the non-collapsed state and the collapsed states.

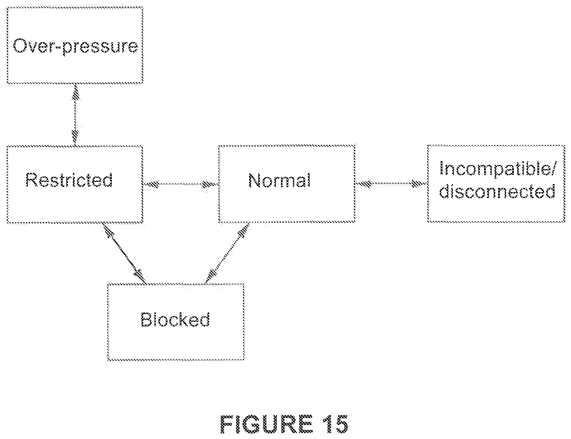

Figure 15 is a state diagram of the states of operation of the respiratory system 22 with reference to the pressure limit curves of Figures 9 to 14. From the normal state of operation of the respiratory system 22, the normal state may transition to the incompatible/disconnected state. In the incompatible/disconnected state, the controller may increase the flow of respiratory gases to approach a target flow rate/set point, but the sensed pressure will not meet expected values, so controller 24 may trigger an alarm to a user of the respiratory system 22 and/or the controller may operate the flow modulator 26 or a proportional valve 25 to reduce or stop the flow.

The normal state may also transition to a restricted state at which system pressure for example meets (but does not exceed) the pressure limit curve C for the corresponding flow rate. That is, in some embodiments, when sensed pressure is at the pressure limit curve C for a given flow rate, the controller initiates pressure-guided control to stay at or return below the pressure limit. In other embodiments, the controller may reduce the speed of a blower or lower the degree openness of a proportional valve of the flow modulator when the sensed pressure meets or exceeds the pressure limit curve C. Alternatively, the controller 24 may remain in pressure-guided control but alter the target flow rate to a lower set point. For example, when pressure meets or exceeds the pressure limit curve, the controller may override the target flow rate of e.g. 70 L/min prescribed by the operator and set a new target flow rate at a lower set point of e.g. 20 L/min. When controller 24 determines the system pressure to have dropped below the pressure limit, the controller may then switch to flow-guided control and increase the speed of a blower or increase the degree openness of a proportional valve of the flow modulator in order to (gradually) meet the flow rate set point. The controller 24 may check system pressure periodically or continuously and, if the system pressure has not reduced below the pressure limit, maintain or further decrease the flow modulator parameters discussed above. A reduced blower speed may be achieved by e.g. reducing a control signal or supply current (or voltage) to the flow modulator to cause decreasing angular velocity of the blower. Alternatively/additionally the controller may control operation of a proportional valve to decrease flows. In other embodiments, the controller 24 may be configured to determine current system pressure only once the flow rate inputs have established that the system is operating at the lower flow rate set point, rather than periodically or continuously determining system pressure.

In the normal state, the controller may control flow modulator 26 to deliver a target flow rate (also referred to as set point flow rate) through the nasal cannula 200 to the patient. The controller 24 may control the respiratory system 22 to allow operation in the restricted state without switching between flow-guided and pressure-guided control to minimise accidental or unnecessary pressure and/or flow limiting events occurring in response to transient or temporary changes in system pressure. This allows users of the respiratory system 22 more freedom to work normally with the respiratory system 22, by allowing moving/bending of circuits, checking if a patient is breathing, moving their head, possibly awakening the patient, conducting nasal fibre optic intubation, etc without causing a transition to the over pressure state described elsewhere. The pressure limit curve C is in some embodiments set to be as high as possible while ensuring the patient is safe.

When the sensed pressure increases to cross pressure limit curve C at a corresponding flow rate, the restricted state transitions to the over pressure state. In the over pressure state, the controller 24 is configured to control the flow modulator 26 or a proportional valve 25 to reduce or stop the flow of respiratory gases as described above and/or to initiate an audible and/or visible an alarm if this state occurs for an extended time period. Ideally transition to the over-pressure state causes the controller to transition to pressure-guided control and in some embodiments, the controller may be configured to sound an alert while the controller is in pressure-guided control mode, or if pressure-guided control persists for longer than a pre-programmed time duration, or a pre-programmed proportion of a time period over which respiratory support has been provided to the patient. Triggering of one or more alerts or alarms while in the over-pressure state may also be contingent on other parameters such as whether there is flow delivered to the patient and/or what proportion (e.g. 90%) of the target flow rate is being achieved.

In some embodiments, the controller is configured to provide an alert system that conveys to the user prioritised information or warnings. For instance, a first visible and/or audible alert may be red and higher volume and/or a continuous tone designating the highest priority, a second visible and/or audible alert may be orange and lower volume and/or a pulsed tone to designate a second priority, a third visible and/or audible alert may be green (or yellow or white etc.) and/or a pulsed tone of lower pulse frequency to designate a third priority and so on. The visible alerts may be presented by an I/O interface 20 or by LEDs or the like provided on housing 33 or on a component that may be located remotely from the housing, e.g. attachable to an IV pole, bed rail or a user, for user convenience. The various priorities may correspond to various system states which have varying degrees of alert priority (based on requirements of patient care). The alerts may be generated by the controller 24 or by any combination of system controller/microcontrollers such as a safety microcontroller which may be configured to provide or allow for multiple levels of brightness of the LEDs (e.g. dim and full bright) to ensure that there is always at least one LED illuminating when the device is receiving power. This ensures that any failure in the system (e.g. crashing of the microcontroller that drives the LEDs) does not indicate incorrectly that the entire system is off. In this arrangement, the alert LEDs will be completely extinguished only when power to the device is completely disconnected.

In yet further embodiments, the controller may be configured to provide different alerts according to where the system is operating on the pressure limit curve C. By way of example, the controller 24 may be configured to emit a first audible alert comprising of e.g. 5 beeps per second from an I/O interface 20 when a blockage is introduced to the system causing the system pressure to meet or exceed the pressure limit curve at or near the target flow rate, triggering controller 24 to switch to pressure-guided control (see X in Figure 9). Alternatively/additionally, the controller 24 may be configured to emit a second audible alert comprising of e.g. 3 beeps per second from the I/O interface 20 when the system pressure travels down the pressure limit curve C (toward and past Y in Figure 9), and a third audible alert comprising of e.g. 1 beep per second may be emitted when the system pressure and/or flow is reduced even further (see Z in Figure 9). The beep rates provided in this example are suggestions made only to illustrate the utility of different alert sounds (or visible cues) representing different operational conditions of the system providing useful feedback to the clinician on e.g. whether or not the collapsible portion 204 has been fully collapsed. It is to be understood that these and other alerts may be provided audibly or visibly by the controller operating the I/O interface 20 and/or other components which may be located remotely from the system housing 33 as described above.

Blocked is a further state of the respiratory system 22 which may be transitioned to from the normal or restricted states. The blocked state is transitioned from the restricted or normal states when the sensed flow rate reaches a threshold of, for example, 2 L/min or reaches 0 L/min and when the system pressure is at or beyond the pressure limit curve C for a corresponding flow rate. In some embodiments, when in the blocked state, the controller 24 controls the flow modulator 26 to modulate the flow of respiratory gases (which may be 0 L/min) to a target pressure above 0 cmH₂O (which is where pressure limit curve C intersects the y-intercept). Hence in such embodiments, there will be a system pressure at zero or substantially low flow rates. When an obstruction or blockage in the system is removed, the system pressure will decrease and the flow rate of the flow of respiratory gases will increase. The controller 24 will therefore control the flow modulator 26 to modulate the flow of respiratory gases to a new higher target pressure that corresponds to the higher flow rate. As the flow modulator 26 increases the pressure of the flow of respiratory gases, the flow rate will accordingly increase and results in a new higher flow rate that establishes a new target pressure that corresponds to the new higher flow rate. Accordingly, the flow modulator 26 is controlled to modulate the flow of respiratory gases to an increasing target pressure until the flow rate set point is met following which the controller 24 switches to flow-guided control. In addition, or in an alternative, the controller 24 determines whether the sensed pressure and flow rate corresponds to pressure waveforms that match when the nasal cannula 31 is collapsed indicating that bag mask ventilation has occurred. If this is not determined, the controller 24 may continue to increase flow and return to the first configuration.

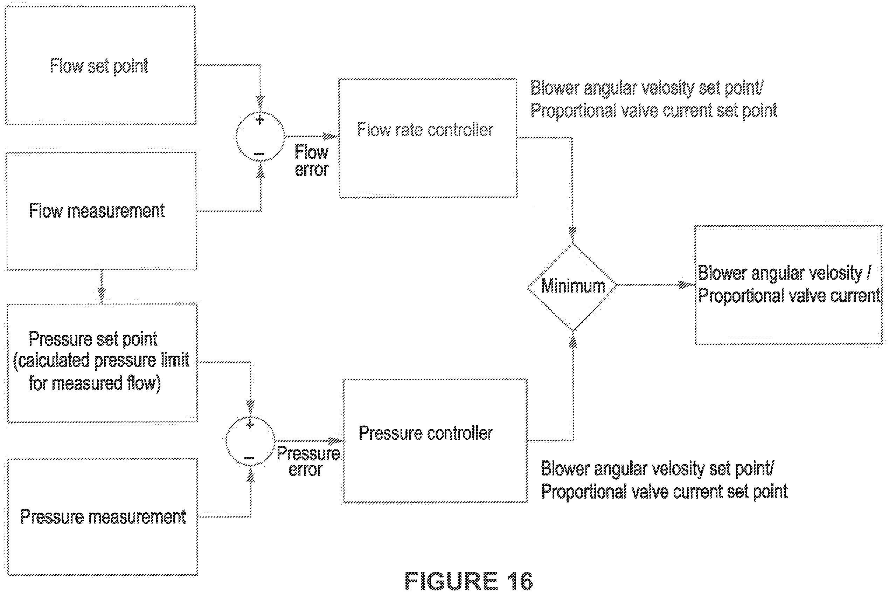

Figure 16 is a high-level schematic representation of operation control of the respiratory system 22 according to an example of the present disclosure. In the example shown, the controller 24 may be considered to provide a flow rate controller providing flow rate control output and a pressure controller providing a pressure control output, operating as separate modules of the controller 24. The flow rate controller uses the flow set point (which may be the target flow rate) and the sensed flow rate (flow measurement) as inputs and the pressure controller uses a pressure set point calculated from the pressure limit curve C for corresponding measured flow rates and the sensed pressure (pressure measurement) as input. The controller 24, implementing these pressure and flow rate controllers concurrently, is thus able to provide pressure-guided control and flow-guided control to the respiratory system 22. Changes in control, in either pressure-guided or flow-guided control mode, may be achieved by altering control of the flow modulator e.g. to achieve a change in blower angular velocity and/or altering control of the proportional valve by changing supply current or voltage to achieve a required increase or decrease in flow rate. As mentioned, the predetermined pressure threshold values and corresponding flow rates represented by curve C may be stored in a memory component of or operatively coupled with controller 24 and represented in any suitable form such as one or more of a function, a curve, a look up table or algorithm or the like.To implement control, the controller 24 selects the minimum control value determined by the flow control system and the pressure control system, for control of the flow modulator/blower or proportional valve.

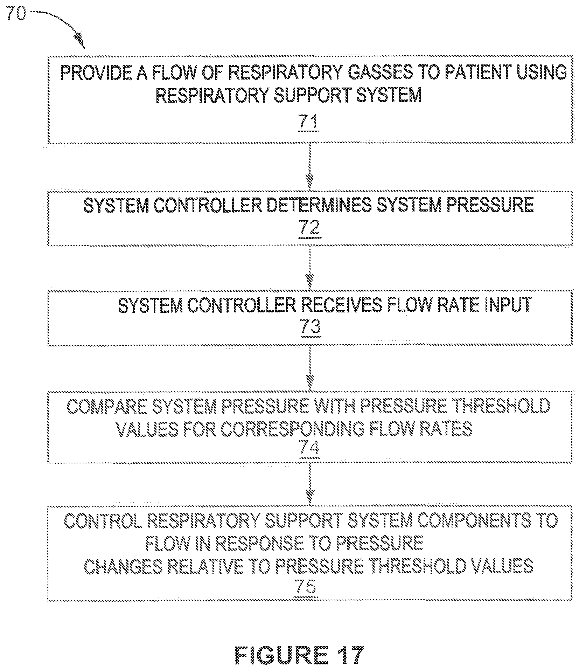

Referring now to Figure 17, there is shown schematically steps in a method 70 of operating a respiratory support system 22, according to embodiments of the present disclosure, for providing a flow of respiratory gases to a patient. In a step 71 a flow of respiratory gases is provided to a patient with the respiratory support system 22 which may be at a target flow rate. This typically involves applying a patient interface to the patient, which receives a flow of respiratory gases from a flow source of the respiratory support system, under the control of a controller of the respiratory support system. In a step 72, the controller determines system pressure corresponding to gases pressure downstream of the flow modulator. The system pressure is typically determined using one or more sensors such as one or more pressure sensors configured to determine pressure of the flow of respiratory gases in the respiratory system although in other embodiments the system pressure may be measured and provided to the controller by manual means such as by an operator entering the values using an input/output interface in operative communication with the controller. In a step 73 the controller receives an input indicative of the flow rate of the flow of respiratory gases in the system. Typically, the flow rate is measured by one or more sensors, such as one or more flow rate sensors. In a step 74 the controller compares the system pressure determined at step 72 with pressure threshold values which have been pre-determined for corresponding flow rates at which the respiratory support system may be operated. In particular, the controller compares the system pressure determined at step 72 with pressure threshold values for the corresponding target flow rate. The pressure threshold values may be stored in a memory component of or operatively coupled with the controller and represented in any suitable form such as one or more of a function, a curve, a look up table or algorithm or the like. In some embodiments the memory component may include a computer-readable medium (e.g., a disk, hard drive, USB, optical drive or other data storage device) containing program instructions for causing the controller to perform the necessary control as described herein. In a step 75, the controller controls components of the respiratory support system, such as a flow modulator to maintain, increase or decrease the flow of respiratory gases in response to system pressure meeting or exceeding, or not meeting or exceeding the pressure threshold values at the target flow rate as determined by the controller in step 74. In embodiments where the comparison in step 74 determines the pressure threshold to have been met or exceeded for the target flow rate, the controller may switch to a pressure-guided control mode (Figure 15) in which the controller controls operation of the system components to reduce flow and/or pressure in the system. Alternatively, the controller may remain in flow-guided control mode but alter the target flow rate to a lower set point. In embodiments where the comparison in step 74 determines the pressure to have been reduced below the pressure threshold and the target flow rate is achieved, the controller switches to a flow-guided control mode (Figure 15). In some embodiments where the comparison in step 74 determines the pressure to have been reduced below the pressure threshold but the target flow rate has not been achieved, the controller may remain in a pressure-guided mode where the controller controls the flow modulator to a target pressure.

It is to be understood that various modifications, additions and/or alternatives may be made to the parts previously described without departing from the ambit of the present invention as defined in the claims appended hereto.

The invention may also be said broadly to consist in the parts, elements and features referred to or indicated in the specification of the application, individually or collectively, in any or all combinations of two or more of said parts, elements or features. Where, in the foregoing description reference has been made to integers or components having known equivalents thereof, those integers are herein incorporated as if individually set forth.

Where any or all of the terms "comprise", "comprises", "comprised" or "comprising" are used in this specification (including the claims) they are to be interpreted as specifying the presence of the stated features, integers, steps or components, but not precluding the presence of one or more other features, integers, steps or components or group thereof.

It is to be understood that the following claims are provided by way of example only and are not intended to limit the scope of what may be claimed in the future. Features may be added to or omitted from the claims at a later date so as to further define or re-define the invention or inventions.

### REPRESENTATIVE FEATURES

1. A respiratory system for providing a flow of respiratory gases to a patient, the system comprising:
   a flow modulator;
   a controller configured to receive an input of a flow rate and a pressure of the flow of respiratory gases in the system, and configured to:
      control the flow modulator to provide the flow of respiratory gases at a target flow rate to a patient; and
      control the flow modulator to modulate the flow of respiratory gases to a target pressure when the pressure of the flow of respiratory gases in the system meets or exceeds a pressure threshold value corresponding to that target flow rate.
2. The respiratory system of clause 1, wherein the target pressure comprises the pressure threshold value corresponding to the target flow rate or a pressure threshold value corresponding to a flow rate less than the target flow rate.
3. The respiratory system of clause 1 or clause 2, wherein the controller is configured to control the flow modulator to modulate the flow of respiratory gases to the target pressure when the flow rate of the flow of respiratory gases is less than the target flow rate.
4. The respiratory system according to any one of the preceding clauses, wherein the controller is configured to control the flow modulator to modulate the flow of respiratory gases to the target flow rate when the flow rate of the flow of respiratory gases is above the target flow rate.
5. The respiratory system according to any one of the preceding clauses, wherein the controller is configured to control the flow modulator to the target pressure when the pressure of the flow of respiratory gases exceeds the pressure threshold value corresponding to the target flow rate or a pressure threshold value corresponding to flow rates less than the target flow rate.
6. The respiratory system according to any one of the preceding clauses, wherein when the system begins operation from an 'off' or dormant state, the controller is configured to monitor the flow rate of the flow of respiratory gases in the system and is configured to control the flow modulator to modulate the flow of respiratory gases to the target flow rate, and optionally wherein the target flow rate changes over time to increase from a current value to a flow rate set point.
7. The respiratory system according to any one of the preceding clauses, wherein the target flow rate is a flow rate set point.
8. The respiratory system according to clause 6 or clause 7, wherein the flow rate set point is determined by a user of the respiratory system.
9. The respiratory system according to any one of the preceding clauses, wherein the controller comprises a flow rate controller providing a flow rate control output and a pressure controller providing a pressure control output, wherein the control input to the flow modulator is the minimum of the flow rate control output and the pressure control output.
10. The respiratory system according to clause 9, wherein the flow modulator comprises a blower, and the flow rate control output and pressure control output comprise an angular velocity of the blower.
11. The respiratory system according to clause 9 or 10, wherein the flow modulator comprises a proportional valve, and the flow rate control output and pressure control output comprise a size of a flow path restriction through the proportional valve.
12. The respiratory system according to any one of the preceding clauses, wherein the controller is configured to receive a flow rate value indicative of flow rate of respiratory gases provided to the patient.
13. The respiratory system according to clause 12, wherein the controller is operable in a first control mode when the flow rate is above the target flow rate to control the flow rate to the target flow rate, and the controller is operable in a second control mode when the pressure of the flow of respiratory gases is above the pressure threshold value corresponding to the target flow rate or is above a or the pressure threshold value corresponding to a flow rate less than the target flow rate, to control the pressure to the target pressure.
14. The respiratory system according to any one of the preceding clauses, comprising one or more flow rate sensors configured to sense a flow rate of the flow of respiratory gases in the system to the patient.
15. The respiratory system according to any one of the preceding clauses, comprising one or more pressure sensors configured to sense a pressure of the flow of respiratory gases in the system to the patient.
16. The respiratory system according to clause 14 or clause 15, wherein the controller is further configured to receive an input indicative of the flow rate of the flow of respiratory gases in the system from the one or more flow rate sensors, and an input indicative of the pressure of the flow of respiratory gases in the system from the one or more pressure sensors.
17. The respiratory system according to any one of the preceding clauses, further comprising a delivery conduit for providing a flow of gases from the flow modulator to a patient interface, wherein the patient interface is configured to deliver the flow of respiratory gases to the patient.
18. The respiratory system according to clause 17, wherein the patient interface comprises a gases delivery side arm in fluid communication with the delivery conduit, a manifold provided at an end of the gases delivery side arm, and one or more nasal elements extending from the manifold, the one or more nasal element configured to provide the flow of respiratory gases to one or more nares of the patient, wherein the gases delivery side member comprises a collapsible portion.
19. The respiratory system of clause 18, wherein the collapsible portion is operable in a first configuration in which the collapsible portion is in a substantially open condition, and in a second configuration in which the collapsible portion is in a substantially closed condition.
20. The respiratory system according to any one of the preceding clauses, wherein when the controller controls the flow modulator to the target pressure, flow to the patient is reduced to a reduced flow rate which is: about 15L/min or less; or about 10L/min or less; or about 10L/min; or about 5L/min to about 10L/min or less than about 5L/min or 0L/min.
21. The respiratory system according to any one of the preceding clauses, wherein the controller is configured to control the flow modulator to increase the flow of respiratory gases towards the target flow rate.
22. The respiratory system of clause 17 or clause 18, wherein the patient interface comprises a nasal cannula, optionally a non-sealing nasal cannula.
23. The respiratory system of any one of clauses 27, 18 and 22, wherein the delivery conduit further comprises a delivery circuit and a patient breathing circuit disposed between the delivery circuit and the patient interface, and wherein the patient breathing circuit is connected to the delivery circuit by an outlet connector.
24. The respiratory system of any one of the preceding clauses, wherein the flow modulator comprises one or more of:
   a flow generator configured to be controlled by the controller to modulate the flow of respiratory gases to a patient; and
   a proportional valve configured to be controlled by the controller to modulate the flow of respiratory gases.
25. The respiratory system of clause 24, wherein the flow generator comprises a blower configured to be controlled by the controller to generate the flow of respiratory gases.
26. The respiratory system according to any one of the preceding clauses, comprising an Oxygen (O₂) pressure sensor configured to sense pressure in an O₂ delivery circuit of the respiratory system.
27. The respiratory system according to any one of the preceding clauses, comprising an Oxygen (O₂) flow rate sensor configured to sense flow rate of a flow of O₂ in an O₂ delivery circuit of the respiratory system.
28. The respiratory system of clause 27 when dependent on clause 26, comprising a proportional valve disposed between the O₂ pressure sensor and the O₂ flow rate sensor in the O₂ delivery circuit.
29. The respiratory system of any one of clauses 26 to 28, when dependent on clause 23, wherein the patient breathing circuit is connected to the O₂ delivery circuit and an air delivery circuit and the patient breathing circuit and/or the patient interface further comprises a patient pressure sensor and a patient flow rate sensor.
30. The respiratory system of clause 29, wherein the flow modulator comprises a blower, and the blower is disposed in the patient breathing circuit before the patient pressure sensor and the patient flow rate sensor.
31. The respiratory system of any one of the preceding clauses, wherein the controller is in operative communication with, or comprises, a memory component storing one or more of a function, a curve, a look up table or algorithm providing a relationship between the pressure threshold values and corresponding flow rates.
32. The respiratory system of clause 31, wherein the controller is configured to control the flow modulator to reduce the flow of respiratory gases by a variable rate of decrease.
33. The respiratory system of clause 31 or clause 32, wherein the relationship between the pressure threshold values and corresponding flow rates may be represented by a pressure limit curve or function defining a curve having a sigmoidal shape.
34. The respiratory system of any one of clauses 31 to 33, wherein the relationship between the pressure threshold values and corresponding flow rates comprises a first pressure region, a second pressure region, and a transition region disposed between the first pressure region and the second pressure region.
35. The respiratory system of clause 34, when dependent on clause 19, wherein the first pressure region corresponds to when the collapsible portion is substantially in the first configuration and the second region corresponds to when the collapsible portion is substantially in the second configuration.
36. The respiratory system of clause 35, wherein the first pressure region comprises pressure threshold values offset from baseline pressure values by a first pressure margin and, the second pressure region comprises pressure threshold values offset from baseline pressure values by a second pressure margin, whereby the first pressure margin is greater than the second pressure margin.
37. The respiratory system of clause 36, wherein the first and second margins provide an offset of the pressure threshold values in the first pressure region which is substantially parallel to an offset of the pressure threshold values in the second pressure region of the pressure limit curve, and the first pressure region and the second pressure region correspond to a minimum rate of change in the flow of respiratory gases.
38. A respiratory system of any one of clauses 34 to 37, wherein the gradient of the transition region of the pressure limit curve corresponds to a maximum rate of change in the flow of respiratory gases.
39. A respiratory system of any one of clauses 34 to 38, wherein the transition region of the pressure limit curve is centred about a transition flow rate.
40. A respiratory system of clause 38, wherein the controller is configured to control the flow modulator to achieve a flow of respiratory gases at the maximum rate of change when the pressure and the corresponding flow rate is in the transition region of the pressure limit curve.
41. A respiratory system of clause 36, wherein the first and the second pressure margins provide a margin for a temporary decrease in system pressure of the flow of respiratory gases from below the pressure threshold values without the controller controlling the flow modulator to increase the flow of respiratory gases.

This divisional application is divided from EP21905962.3 (the 'parent application') and the divisional specification as filed comprises the content of the parent application, including the original claims recited as 'representative features' above. The scope of this disclosure therefore includes the full content of the parent application. In any case, protection may be sought for any features disclosed in the parent application as filed.

## Claims

1. A respiratory system for providing a flow of respiratory gases to a patient, the system comprising:
a controller; and
a flow modulator configured to be controlled by the controller to provide a flow of respiratory gases to a patient;
wherein the controller is configured to control the flow modulator to a flow rate determined pressure set point; and
wherein the controller is configured to control the flow modulator to limit the flow rate of the flow of respiratory gases up to and including a flow rate set point.

2. The respiratory system of claim 1, wherein the flow rate set point is set by a user of the respiratory system.

3. The respiratory system of claim 1 or claim 2, wherein the flow rate set point is set by a user providing an input to the controller.

4. The respiratory system of any one of claims 1 to 3, wherein the system comprises a plurality of flow rate determined pressure set points, to which the controller is configured to control the flow modulator.

5. The respiratory system of claim 4, wherein one or more of the plurality of flow rate determined pressure set points is based on the flow rate of the flow of respiratory gases.

6. The respiratory system of claim 4 or claim 5, wherein one or more of the flow rate determined pressure set point is predetermined.

7. The respiratory system of any one of claims 4 to 6, wherein the flow rate determined pressure set point when the flow rate of the flow of respiratory gases is at or close to the flow rate set point, is separated by a first margin from a normal system pressure value, wherein the flow rate determined pressure set point when the flow rate of the flow of respiratory gases at or close to 0L/min, is separated by a second margin from a normal system pressure value, wherein the first margin is greater than the second margin.

8. The respiratory system of claim 7, wherein the flow rate determined pressure set point when the flow rate of the flow of respiratory gases at or close to 0L/min, is 0cmH2O.

9. The respiratory system of any one of claims 1 to 8, wherein the flow modulator comprises a blower and/or a proportional valve.

10. The respiratory system of any one of claims 1 to 9, wherein the respiratory system further comprises one or more of a flow sensor, pressure sensor and a user interface.

11. The respiratory system of any one of claims 1 to 10, wherein respiratory system further comprises a non-sealing nasal interface in fluid communication with the flow modulator and configured to provide the flow of respiratory gases to the patient.

12. The respiratory system of claim 11, wherein the non-sealing nasal interface comprises a gases delivery side arm, a manifold provided at an end of the gases delivery side arm, and one or more nasal elements extending from the manifold, the one or more nasal element configured to provide the flow of respiratory gases to one or more nares of the patient, wherein the gases delivery side member comprises a collapsible portion.

13. The respiratory system of claim 12, wherein the collapsible portion is operable in a first configuration in which the collapsible portion is in a substantially open condition, and in a second configuration in which the collapsible portion is in a substantially closed condition.

14. The respiratory system of any one of claims 1 to 13, wherein the flow rate set point is more than 0 L/min, optionally more than 0 L/min to about 120 L/min.

15. The respiratory system of any one of claims 1 to 13, wherein the flow rate set point is between about 20 L/min to about 90 L/min, optionally about 40 L/min to about 70 L/min.
